# EUROPEAN PATENT SPECIFICATION

(11) **EP 4 019 616 B1**
(45) Date of publication and mention of the grant of the patent: **28.01.2026**
(21) Application number: 20383158.1
(22) Date of filing: 24.12.2020
(51) Int. Cl.: C11D 1/72, A61K 8/00, A61Q 5/00, A61Q 5/12, C11D 1/722, C11D 3/00, C11D 3/20, C11D 3/30, C07C 213/06, C07C 213/08, C11D 1/62, A61K 8/34, A61K 8/37, A61K 8/41

(54) **QUATERNARY ESTER AMMONIUM COMPOUND COMPOSITIONS**
QUATERNÄRE ESTERAMMONIUMVERBINDUNGSZUSAMMENSETZUNGEN
COMPOSITIONS DE COMPOSÉ D'AMMONIUM ESTER QUATERNAIRE

(43) Date of publication of application: 29.06.2022
(73) Proprietor: KAO CORPORATION, S.A., 08210 Barberà del Vallès, Barcelona (ES)
(72) Inventor: PI BOLEDA, Bernat, 08210 Barberà del Vallès (Barcelona) (ES); PEY GUTIÉRREZ, Carmen M., 08210 Barberà del Vallès (Barcelona) (ES); SOBREVIAS ALABAU, Jaume, 08210 Barberà del Vallès (Barcelona) (ES); NOGUÉS LÓPEZ, Blanca, 08210 Barberà del Vallès (Barcelona) (ES)
(74) Representative: Hoffmann Eitle

(56) References cited:
- EP-A1- 3 418 353
- WO-A1-2020/014659
- US-A- 5 783 534

## Description

### FIELD OF THE INVENTION

The invention is set out in the appended set of claims. The present disclosure relates to compositions comprising a combination of a quaternary ester ammonium compound, a fatty acid ester and a fatty alcohol, wherein the fatty acid ester comprises a fatty acid ester derived from a linear fatty alcohol, and a fatty acid ester derived from a branched fatty alcohol and/or a branched fatty acid; and methods of making the composition.

The present disclosure also relates to fabric softener compositions comprising the previously described compositions, and methods of making and using the fabric softener compositions.

The present disclosure also relates to hair conditioner compositions comprising the previously described compositions, and methods of making and using the hair conditioner composition.

### STATE OF THE ART

Compositions comprising quaternary ester ammonium compounds (esterquats) are generally understood by those skilled in the art to be compositions comprising quaternized fatty acid alkanolamine ester salts which are typically used for softening fibers and for conditioning hair.

Compositions comprising quaternary ester ammonium compounds are used in different applications due to their relevant properties. For instance, they are used in fabric softener applications due to their softening performance, their biodegradability, reasonably low aquatic toxicity, as well as their storage stability, their hydrolysis stability in aqueous dispersions with little change in dispersion viscosity, appropriate provision of lubricity in fabrics or appropriate compatibility with other components including perfumes.

Compositions comprising quaternary ester ammonium compounds are also of special interest for hair conditioning applications due to its characteristics for providing good performance in terms of combing force, shine, volume, body, elasticity and manageability, as well as appropriate lubricity of hair fibers and antistatic effect.

There is a need in the art for a quaternary ester ammonium composition, in particular for use as a fabric softening composition and as a hair conditioning composition, which deliver good results in terms of associated performances for such fabric softening and hair conditioning application.

There are several attempts in the current state of the art aimed to provide solutions to the cited above problems and compositions.

WO2012072368 proposes fabric conditioner compositions comprising (a) from 2 to 9 wt. % of a fabric softening active, wherein the fabric softening active is an ester-linked quaternary ammonium compound having fatty acid chains comprising from 20 to 35 wt. % of saturated C₁₈ chains and from 20 to 35 wt. % of monounsaturated C₁₈ chains, and (b) from 0.05 to 1.0 wt. % of a hydrophobic agent having a ClogP of from 4 to 9; wherein the aqueous fabric conditioner composition has a stable viscosity.

WO200159052 proposes a fabric conditioning composition comprising a cationic fabric softening compound dispersed in water, wherein the composition also comprises at least one salt of a multivalent inorganic anion or non-sequestering organic multivalent anion. Reported results describe an improved viscosity stability values of the system. It is described that the invention is particularly suitable for use with quaternary ammonium compound comprising a two C₁₂₋₁₈ alkyl or alkenyl groups connected to the molecule via at least one ester link. Preferably the mixture comprises sodium chloride and sodium sulphate as salts.

US20030161808 discloses quaternary ester ammonium mixtures, containing quaternary ester ammonium compounds with an acyl component derived from C₆-C₁₈ fatty acids and quaternary ester ammonium compounds with an acyl component derived from C₁₈-C₂₂ fatty acids. Cosmetic compositions are prepared with the esterquat mixtures and results on hair combability and flexural strength are reported.

EP 3 418 353 A1 discloses a fabric softener active composition comprising: a component (a), said component being at least one or a mixture of quaternary mono-, di- or tri-ester ammonium compounds; a component (b), said component being a fatty acid ester or a mixture of fatty acid esters, wherein the fatty acid ester is derived from a C3-C10 fatty alcohol or a mixture of C3-C10 fatty alcohols and wherein the component (b) content is in the range from 5 to 50% wt. based on the total weight of the fabric softener active composition; a component (c), said component being a fatty acid or a mixture of fatty acids, wherein the component (c) content is higher than 0 and up to 15% wt. based on the total weight of the fabric softening active composition.

US 5,783,534 A discloses a process for the production of solid esterquats, in which fatty acid triethanolamine esters corresponding to formula (I) are quaternized in known manner with alkylating agents in the presence of a) fatty alcohol polyglycol ethers and b) fatty acid partial glycerides.

WO 2020/014659 Al discloses esterquat compositions that comprise, based on the weight of the composition, 0% to about 70% by weight of a solvent, and about 30% to 100% by weight of a mixture comprising about 50% to about 80% by weight, based on the weight of the mixture, of esterquats and about 20% to about 50% by weight, based on the weight of the mixture, of mono- and diglycerides.

There is however still a need for compositions comprising quaternary ester ammonium compounds that meet the requirements of compositions for the care of fabrics or the conditioning of hair fibers.

The present disclosure aims at addressing the above-described needs, and provides a composition comprising a quaternary ester ammonium compound, a fatty acid ester and a fatty alcohol, wherein the fatty acid ester comprises a fatty acid ester derived from a linear fatty alcohol and a fatty acid ester derived from a branched alcohol and/or a branched fatty acid, a process of preparation of said composition; and its use in fabric softener applications and/or in hair conditioner applications. The compositions are able to comply with the requirements imposed on them and provide good performance.

### SUMMARY OF THE INVENTION

The first object of the present invention is a composition comprising:
i. component (a) which comprises at least one or more quaternary mono-, di-, or tri-ester ammonium compounds; and
ii. component (b), said component being a fatty acid ester mixture, comprising:
   - at least a component (b1) being a fatty acid ester or a mixture of fatty acid esters of formula (II),

      R₅-COO-R₆ Formula (II)

      wherein R₅ represents a fatty acid moiety being a linear alkyl containing 5 to 23 carbon atoms, or a linear alkenyl group containing 5 to 23 carbon atoms and from 1 to 3 double bonds; R₆ represents an alkyl or alkenyl group derived from a linear alcohol containing 6 to 24 carbon atoms; and
   - at least a component (b2) being a fatty acid ester or a mixture of fatty acid esters of formula (III),

      R₇-COO-R₈ Formula (III)

      wherein R7 represents a fatty acid moiety being a linear or branched alkyl containing 5 to 23 carbon atoms, or a linear or branched alkenyl containing 5 to 23 carbon atoms and from 1 to 3 double bonds; R8 represents an alkyl or alkenyl group derived from a linear or branched alcohol containing 3 to 24 carbon atoms; with the proviso that at least one of R₇ and R₈ represents a branched alkyl or alkenyl group; and
iii. component (c), said component being a fatty alcohol or a mixture of fatty alcohols of formula (IV),

   R₉-O-(CH₂CH₂O)ₗ-(CH₂CHR₁₀O)ₖ-H Formula (IV)

   wherein R₉ represents an alkyl or alkenyl group containing 6 to 24 carbon atoms, R₁₀ represents an alkyl group containing 1 to 4 carbon atoms; 1 represents a number within the range of 0 to 10, k represents a number within the range of 0 to 10, and the sum of l+k represents the average alkoxylation degree which corresponds to a number from 0 to 20, preferably from 0 to 10, more preferably from 0 to 5, and
wherein the composition comprises, in the indicated amounts expressed as percentage by weight with respect to the total weight of the composition:
0.6 to 20.0% of component (b1),
1.0 to 29.0% of component (b2), and
0.4 to 9.0% of component (c).

Another object of the present disclosure is the process for producing said composition.

Another object of the present disclosure is a fabric softening composition comprising said previously described composition. A process of preparation of said fabric softening composition is also an object of the present disclosure. Another object of the present disclosure is a method to soften and condition textiles or fabrics by applying the fabric softener composition to fabrics or textiles. The use of the fabric softening composition for the softening of fabrics is another object of the present disclosure.

Another object of the present disclosure is a hair conditioner composition comprising said previously described composition. It is also an object of the present disclosure a process of preparation of the hair conditioner composition. Another object of the present disclosure is the use of the hair conditioning composition according to the present disclosure for the conditioning treatment of hair. A method of conditioning hair with the hair conditioning composition, is also an object of the present disclosure.

### DETAILED DESCRIPTION OF THE INVENTION

### QUATERNARY ESTER AMMONIUM COMPOSITION

The main object of the present invention is a composition comprising:
- component (a), which comprises at least one or more quaternary mono-, di- or tri-ester ammonium compounds; and
- component (b), said component being a fatty acid ester mixture, comprising
   i. at least a component (b1) being a fatty acid ester or a mixture of fatty acid esters of formula (II),

      R₅-COO-R₆ Formula (II)

      wherein R₅ represents a fatty acid moiety being a linear alkyl containing 5 to 23 carbon atoms, or a linear alkenyl group containing 5 to 23 carbon atoms and from 1 to 3 double bonds; R₆ represents an alkyl or alkenyl group derived from a linear alcohol containing 6 to 24 carbon atoms; and
   ii. at least a component (b2), said component being a fatty acid ester or a mixture of fatty acid esters of formula (III),

      R₇-COO-R₈ Formula (III)

      wherein R₇ represents a fatty acid moiety being a linear or branched alkyl containing 5 to 23 carbon atoms, or a linear or branched alkenyl containing 5 to 23 carbon atoms and from 1 to 3 double bonds; R₈ represents an alkyl or alkenyl group derived from a linear or branched alcohol containing 3 to 24 carbon atoms; with the proviso that at least one of R₇ and R₈ represents a branched alkyl or alkenyl group; and
- component (c), said component being a fatty alcohol or a mixture of fatty alcohols of formula (IV),

   R₉-O-(CH₂CH₂O)ₗ-(CH₂CHR₁₀O)ₖ-H Formula (IV)

   wherein R₉ represents an alkyl or alkenyl group containing 6 to 24 carbon atoms, R₁₀ represents an alkyl group containing 1 to 4 carbon atoms; 1 represents a number within the range of 0 to 10, k represents a number within the range of 0 to 10, and the sum of l+k represents the average alkoxylation degree which corresponds to a number from 0 to 20, preferably from 0 to 10, more preferably from 0 to 5, and
wherein the composition comprises, in the indicated amounts expressed as percentage by weight with respect to the total weight of the composition:
0.6 to 20.0% of component (b1),
1.0 to 29.0% of component (b2), and
0.4 to 9.0% of component (c).

### (a): Quaternary ester ammonium compound

The composition of the present invention comprises a component (a), said component comprising at least one or more quaternary mono-, di- or tri-ester ammonium compounds (commonly known as mono-esterquat (mono-EQ), di-esterquat (di-EQ), tri-esterquat (tri-EQ)). Preferably, the content of quaternary ester ammonium compounds in the composition is in the range from 70 to 95% wt., more preferably from 75 to 90% wt. based on the total weight of the composition.

In an embodiment of the present invention, the component (a) comprises, at least one quaternary mono-ester ammonium compound, at least one quaternary di-ester ammonium compound, and at least one quaternary tri-ester ammonium.

In an embodiment of the present invention, said component (a) comprises at least one or more quaternary mono-, di-, or tri-ester ammonium compounds of formula (I1), (I2), (I3): 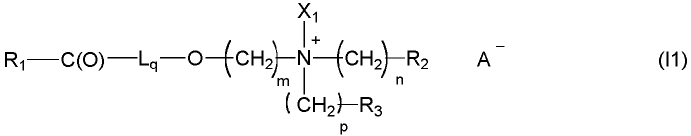 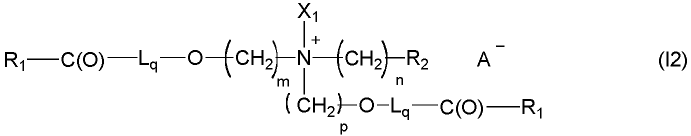 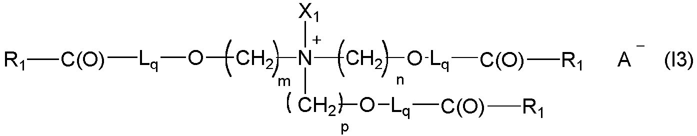 wherein in formulae (I1), (I2), (I3):
R₂ and R₃ each independently represent -H or -OH;
X₁ represents a hydroxyalkyl group containing 1 to 4 carbon atoms, an alkyl group containing 1 to 4 carbon atoms or an alkyl group containing one aromatic group; and
R₁ is a linear or branched alkyl containing 5 to 23 carbon atoms, preferably 11 to 21 carbon atoms, or a linear alkenyl group containing 5 to 23 carbon atoms, preferably 11 to 21 carbon atoms and from 1 to 3 double bonds.
In formulae I1, I2 and I3, each R₁ can independently represent the same or different linear or branched alkyl chain;
A⁻ represents an anion;
L represents a -(OCH₂CH₂)ₐ-(OCHR₄CH₂)_{b}- group, wherein R₄ represents an alkyl group containing 1 to 4 carbon atoms, a represents a number within the range of 0 to 20, b represents a number within the range of 0 to 6, and the sum of a+b represents the average alkoxylation degree which corresponds to a number from 0 to 26; and
m, n, p each independently represents a number within the range from 1 to 4, q represents a number within the range from 0 to 26.

The quaternary ester ammonium compounds of the invention can be ethoxylated and/or propoxylated, since a and b can be larger than 0.

The order of sequence of the ethylene oxide and propylene oxide groups is not critical for the invention.

In the case q is 2 or larger, each L group may be the same or different. Also the (L)_{q} groups contained in the different branches within the compounds of formula (I1), (I2), (I3) may independently represent different meanings.

The sum of a+b preferably represents the average alkoxylation degree which corresponds to a number from 0 to 10, more preferably from 0 to 6, most preferred is 0.

Preferably, X₁ is an alkyl group; more preferably X₁ is a methyl group.

Preferably, A⁻ is selected from a halide, phosphate and alkylsulphate.

Within the present patent application, when a numerical range is indicated, all the individual numbers included in said range are intended to be included. The same shall apply to any other range indicated.

In an embodiment of the present invention, the component (a) comprises or consists of at least one quaternary mono-ester ammonium compound of formula (I1), at least one quaternary di-ester ammonium compound of formula (I2), and at least one quaternary tri-ester ammonium compound of formula (I3).

In another embodiment of the present invention, the component (a) comprises or consists of at least one quaternary mono-ester ammonium compound of formula (I1), at least one quaternary di-ester ammonium compound of formula (I2), and at least one quaternary tri-ester ammonium compound of formula (I3), wherein m=n=p; R₁-C(O)- is a linear acyl group wherein R₁ is a linear alkyl or a linear alkenyl containing from 11 to 21 carbon atoms, preferably derived from (hydrogenated and/or non-hydrogenated) tallow fatty acid or palm fatty acid; R₂ and R₃ each represent -OH, q is 0 (i.e. the compound is not alkoxylated); X₁ is a methyl group; and A⁻ is selected from a halide, phosphate and alkylsulphate, preferably alkylsulphate.

In another embodiment of the present invention, the component (a) comprises or consists of at least one or more quaternary mono-, di- or tri-ester ammonium compounds represented by formula (I1), (I2), (I3) as defined above, wherein R₂ and R₃ independently represent -OH; each m, n, p represents number 2.

The rest of variables have the meanings as indicated above for formula (I1), (I2), (I3).

In another embodiment of the present invention, R₁ is a linear or branched alkyl containing 5 to 23 carbon atoms or a linear alkenyl group containing 5 to 23 carbon atoms and from 1 to 3 double bonds; preferably, the alkyl or alkenyl group contains from 11 to 21 carbon atoms.

As used herein, the term "alkyl" refers to a straight or branched hydrocarbon chain containing from 1 to 23, preferably from 5 to 23 carbon atoms.

As used herein, the term "alkenyl" refers to a linear hydrocarbon chain containing from 2 to 23, preferably 5 to 23 carbon atoms and from one to 3 unsaturations.

As used herein, unless specified otherwise, the term "quaternary ammonium compound" refers to a quaternized (i.e., cationic) nitrogenated species together with a corresponding counterion (i.e., an anion) in such molar ratio that the overall compound is electroneutral (i.e., the overall number of positive charges of the quaternized nitrogenated species is equal to the overall number of negative charges of the counterions).

Linear or branched alkyl or linear alkenyl groups, such as R₁, R₅, R₇ or R₉, can originate from fatty acids, or methyl esters/triglycerides thereof, or can be alkyls or alkenyls derived from oils and fats from plants and/or animals, such as palm, palm kernel, coconut, rapeseed, sunflower, soybean, olive, canola, tall or tallow, possibly totally or partially hydrogenated and purified. Synthetic fatty acids, or methyl esters/triglycerides thereof, such as palmitoleic acid, oleic acid, elaidinic acid, petroselinic acid, linoleic acid, linolenic acid, stearic acid, myristic acid, gadoleic acid, behenic acid and erucic acid, or mixtures thereof, can also be employed in the present disclosure. Preferably, the linear or branched alkyl or linear alkenyl groups proceed from fatty acids derived from palm oil, coconut oil, tallow and hydrogenated tallow, more preferably from tallow or palm and hydrogenated tallow or palm.

The fatty acid is preferably a C₁₂-C₂₂ acid containing a degree of unsaturation such that the iodine value ("IV") is in the range from 0 to 100, preferably from 10 to 90, more preferably in the range from 15 to 85, most preferably 15 to 55. The iodine value can be determined according to EN 14111:2003.

The fatty acid employed in the present disclosure have a cis to trans isomer ratio from 80:20 to 95:5. Preferably the trans isomer content of said fatty acid is less than 10 mol-%.

As used herein the term "alkyl or alkenyl group originating from a fatty acid", refers to the carbon atoms in the alkyl or alkenyl group bonded to the carbonyl group in the respective fatty acid, e.g., an "alkyl group originating from a fatty acid" refers to a C₁₇ alkyl group.

As used herein, the term "alkyl group containing one aromatic group" refers to the alkyl group as defined above, substituted by one aromatic group, wherein "aromatic group" refers to an aryl or heteroaryl group.

"Aryl" refers to aromatic ring systems comprising 6 to 14 carbon atoms, more particularly 6 to 10, even more particularly 6 carbon atoms. Examples of aryl groups are phenyl, naphthyl, indenyl, phenanthryl or anthracyl radical, preferably phenyl or naphthyl radical. Said aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.

"Alkoxy" refers to an alkoxy group as defined above bonded to an oxygen atom (R-O-).

Examples of halogen atoms are Br, Cl, I and F.

The term "heteroaryl" means a monocyclic- or polycyclic aromatic ring comprising carbon atoms, hydrogen atoms, and one or more heteroatoms, preferably, 1 to 3 heteroatoms, independently selected from nitrogen, oxygen, and sulfur. The heteroaryl group has 3 to 15 members and preferably 4 to 8 members. Illustrative examples of heteroaryl groups include, but are not limited to, pyridinyl, pyridazinyl, pyrimidyl, pyrazyl, triazinyl, pyrrolyl, pyrazolyl, imidazolyl, (1,2,3,)- and (1,2,4)-triazolyl, pyrazinyl, pyrimidinyl, tetrazolyl, furyl, thienyl, isoxazolyl, thiazolyl, phenyl, isoxazolyl, and oxazolyl. A heteroaryl group can be unsubstituted or substituted with one or two suitable substituents such as hydroxy, mercapto, halo, alkyl, phenyl, alkoxy, haloalkyl, nitro, cyano, dialkylamino, aminoalkyl, acyl and alkoxycarbonyl, as defined herein. Preferably, a heteroaryl group is a monocyclic ring, wherein the ring comprises 2 to 5 carbon atoms and 1 to 3 heteroatoms.

### Preparation of quaternary ester ammonium compound:

The component (a) comprises at least one or more quaternary mono-, di- or tri-ester ammonium compounds according to the invention. The component (a) can be prepared by i) esterification, reacting a fat source, preferably a fatty acid or a methyl ester/triglyceride thereof, with an alkanolamine (for example, but not limited to, triethanolamine, methyldiethanolamine or dimethylethanolamine) to obtain a mixture containing an esteramine, and ii) subsequently quaternizing the mixture with an alkylating agent.

### i) Esterification step:

It is preferred that the fat source employed in the esterification step is a fatty acid or a mixture of fatty acids. In case a fatty acid methyl ester or a fatty acid triglyceride is used, the transesterification conditions are those described in the state of the art.

The reaction between the fatty acid and the alkanolamine is an esterification which leads to the formation of an esteramine or a mixture of esteramines, and it may be conducted in a known way, as described for example in document ES-A-2021900. Preferably the esterification reaction is carried out at a temperature between 150°C and 200°C for a period of 2-10 hours, preferably at a reduced pressure of about 5 to 200 mbar and in the presence of one of the catalysts known for the esterification such as hypophosphorous acid or paratolueneslfonic acid, and also in the presence of any of the usual stabilizers and antioxidants such as tocopherols, BHT, BHA, etc.

In an embodiment of the present invention, a fatty alcohol or a mixture of fatty alcohols is additionally added to the system in the esterification step. A part of the fatty acid present in the system may react with the fatty alcohol resulting in a fatty acid fatty alcohol ester, as a further product of the esterification step, formed in addition to the esteramine/s.

Suitable fatty alcohol is a C₆ to C₂₄ alcohol or alkoxylated alcohol, preferably a C₁₂ to C₂₀ alcohol or alkoxylated alcohol, more preferably a to C₂₀ alcohol or alkoxylated alcohol.

In an embodiment of the present invention, the C₆ to C₂₄ fatty alcohol is a linear fatty alcohol. As used herein, the term "linear fatty alcohol" refers to a fatty alcohol comprising straight hydrocarbon chain with no pendant hydrocarbon chain attached to the main chain.

Suitable linear fatty alcohols include capryl alcohol (1-octanol), pelargonic alcohol (1-nonanol), capric alcohol (1-decanol), lauryl alcohol (1-dodecanol), lauryl alcohol (1-dodecanol), myristil alcohol (1-tetradecanol), cetyl alcohol (1-hexadecanol), stearyl alcohol (1-octadecanol), elaidyl alcohol (9E-octadecen-1-ol), oleyl alcohol (cis-9-octadecen-1-ol), linoleyl alcohol (9Z, 12Z-octadecadien-1-ol), elaidolinoleyl alcohol (9E, 12E-octadecadien-1-ol), linolenyl alcohol (9Z, 12Z, 15Z-octadecatrien-1-ol), elaidolinolenyl alcohol (9E, 12E, 15-E-octadecatrien-1-ol), arachidyl alcohol (1-eicosanol), behenyl alcohol (1-docosanol), and erucyl alcohol (cis-13-docosen-1-ol) and mixtures thereof.

In an embodiment of the present invention, the linear fatty alcohol has the following formula (V):

R₁₁-O-(CH₂CH₂O)ₛ-(CH₂CHR₁₂O)ₜ-H Formula (V)

wherein R₁₁ represents a linear alkyl or alkenyl group, containing 6 to 24 carbon atoms, preferably from 12 to 20 carbon atoms, more preferably from 18 to 20 carbon atoms; R₁₂ represents an alkyl group containing 1 to 4 carbon atoms; s represents a number within the range of 0 to 10, t represents a number within the range of 0 to 10, and the sum of s + t represents the average alkoxylation degree which corresponds to a number from 0 to 20, preferably from 0 to 10, more preferably from 0 to 5.

The linear fatty alcohol can be ethoxylated and/or propoxylated, since s and t can be larger than 0. The order of sequence of the ethylene oxide and propylene oxide groups is not critical for the invention.

In an embodiment of the present invention the linear fatty alcohol of formula (V) is not alkoxylated.

In an embodiment of the present invention, the linear C₆ to C₂₄ fatty alcohol is additionally added to the system in the esterification step. A part of the fatty acid present in the system may react with the C₆ to C₂₄ linear fatty alcohol resulting in a fatty acid fatty alcohol ester, as a further product of the esterification step, formed in addition to the esteramine/s.

The molar ratio of fatty acid to alkanolamine is from 1.4:1 to 2.5:1, preferably from 1.5:1 to 2.2:1.

The product resulting from the esterification reaction comprises at least one or more mono-, di- and tri-esters of fatty acids. The product may also contain free alkanolamine, free fatty acid, fatty acid fatty alcohol ester and free fatty alcohol. The progress of the reaction may be monitored by non-aqueous potentiometric titration with KOH.

### ii) Quaternization step:

The quaternization of the esterification reaction product of alkanolamine with the fatty acid is conducted in a known way, as described for example in WO-A-9101295. Preferred alkylating agents include, but are not limited to, methyl chloride, dimethyl sulphate or mixtures thereof.

The quaternization may take place in bulk or in solvent, at temperatures ranging from 40°C to 90°C. If an added solvent is employed, then the starting materials and/or product must be soluble in the solvent to the extent necessary for the reaction (possible solvents can be the same solvents as used as component (b) and component (c) as defined below).

The composition that results from the quaternization process comprises quaternized ester compounds having one (monoesterquat), two (diesterquat) or three (triesterquat) ester groups. The product may also contain quaternised alkanolamine, unreacted alkanolamine, unquaternised esteramine-based compounds having one, two or three ester groups. unreacted fatty acid, fatty acid alkyl ester such as fatty acid methyl ester or fatty acid ethyl ester, as well as fatty alkyl methyl ether and fatty alkyl ethyl ether. It may also comprise the solvent, unreacted fatty alcohol and fatty acid fatty alcohol ester.

In an embodiment of the present invention, the content of nitrogenated species in the composition is in the range from 70 to 95% wt., preferably from 75 to 90% wt., based on the total weight of the composition. Nitrogenated species comprise quaternized ester ammonium compounds quaternized alkanolamine, unreacted alkanolamine as well as unquaternized esteramine based compounds having one, two or three ester groups.

In an embodiment of the present invention, the component (a), said component comprising at least one or more quaternary mono-, di-, or tri-ester ammonium compounds of formula (I1), (I2), (I3), is obtained from an esteramine mixture obtained by esterification of triethanolamine and tallow and/or hydrogenated tallow fatty acid and/or palm fatty acid followed by quaternization.

In another embodiment of the present invention, the component (a), said component comprising at least one or more quaternary mono-, di-, or tri-ester ammonium compounds of formula (I1), (I2), (I3), is obtained from an esteramine mixture obtained by esterification of triethanolamine and tallow and/or hydrogenated tallow fatty acid and/or palm fatty acid followed by quaternization, wherein a linear fatty alcohol is additionally added to the system in the esterification step. A part of the fatty acid present in the system may react with the linear fatty alcohol resulting in a linear fatty acid fatty alcohol ester, as a further product of the esterification step, formed in addition to the esteramine/s.

The quaternization may take place in a degree from 60 to 95 mol-% of the totality of the reaction.

Preparation of the component (a) is carried out under conditions according to the person skilled in the art to obtain a mixture of at least one or more quaternary mono-, di- or tri-ester ammonium compounds of formula (I1), (I2), (I3).

In an embodiment of the present invention, the component (a) comprises at least one or more quaternary mono-, di- or tri-ester ammonium compounds of formula (I1), (I2), (I3), wherein m=n=p; R₁-C(O)- is a linear acyl group wherein R₁ is an alkyl or alkenyl group containing from 11 to 21 carbon atoms, preferably derived from (hydrogenated or non-hydrogenated) tallow fatty acid or palm fatty acid; R₂ and R₃ each represent -OH, q is 0 (i.e. the compound is not ethoxylated); X₁ is a methyl group; and A⁻ is selected from a halide, phosphate and alkylsulphate, preferably alkylsulphate. Such compound may be produced by esterifying (hydrogenated and/or non-hydrogenated) tallow fatty acid or palm fatty acid and triethanolamine, wherein the ratio of tallow fatty acid or palm fatty acid to triethanolamine is from 1.5:1 to 2.2:1, and subsequently methylating the esteramine obtained thereby.

In another embodiment of the present invention, the component (a) comprises at least one or more quaternary mono-, di- or tri-ester ammonium compounds of formula (I1), (I2), (I3), wherein m=n=p; R₁-C(O)- is a linear acyl group wherein R₁ is an alkyl or alkenyl group containing from 11 to 21 carbon atoms, preferably derived from (hydrogenated or non-hydrogenated) tallow fatty acid or palm fatty acid; R₂ and R₃ each represent -OH, q is 0 (i.e. the compound is not ethoxylated); X₁ is a methyl group; and A⁻ is selected from a halide, phosphate and alkylsulphate, preferably alkylsulphate. Such compound may be produced by esterifying (hydrogenated and/or non-hydrogenated) tallow fatty acid or palm fatty acid and triethanolamine, wherein the ratio of tallow fatty acid or palm fatty acid to triethanolamine is from 1.5:1 to 2.2:1, and subsequently methylating the esteramine obtained thereby; wherein a linear fatty alcohol of formula (V) is added to the system in the esterification step. A part of the (hydrogenated and/or non-hydrogenated) tallow fatty acid or palm fatty acid may react with the linear fatty alcohol resulting in a linear fatty acid fatty alcohol ester, as a further product of the esterification step, formed in addition to the esteramine/s.

### (b): Fatty acid ester

The composition of the present invention comprises a component (b), said component being a fatty acid ester mixture comprising:
- at least a component (b1) being a fatty acid ester or a mixture of fatty acid esters of formula (II),

   R₅-COO-R₆ Formula (II)

   wherein R₅ represents a fatty acid moiety being a linear alkyl containing 5 to 23 carbon atoms, preferably 11 to 21 carbon atoms, or a linear alkenyl group containing 5 to 23 carbon atoms, preferably 11 to 21 and from 1 to 3 double bonds; R₆ represents an alkyl or alkenyl group derived from a linear fatty alcohol containing from 6 to 24 carbon atoms, preferably from 12 to 20 carbon atoms, more preferably from 18 to 20; and
- at least a component (b2) being a fatty acid ester or a mixture of fatty acid esters of formula (III),

   R₇-COO-R₈ Formula (III)

   wherein R₇ represents a fatty acid moiety being a linear or branched alkyl containing 5 to 23 carbon atoms, preferably 11 to 21 carbon atoms, or a linear or branched alkenyl containing 5 to 23 carbon atoms, preferably 11 to 21 carbon atoms, and from 1 to 3 double bonds; R₈ represents an alkyl or alkenyl group derived from a linear or branched fatty alcohol containing 3 to 24 carbon atoms, preferably 3 to 10 carbon atoms; with the proviso that at least one of R₇ and R₈ represents a branched alkyl or alkenyl group and
wherein the composition comprises, in the indicated amounts expressed as percentage by weight with respect to the total weight of the composition:
0.6 to 20.0% of component (b1), and
1.0 to 29.0% of component (b2).

### Component (b1)

The component (b1) is a fatty acid ester or a mixture of fatty acid esters of formula (II):

R₅-COO-R₆ Formula (II)

wherein R₅ represents a fatty acid moiety being a linear alkyl containing 5 to 23 carbon atoms, preferably 11 to 21 carbon atoms, or a linear alkenyl group containing 5 to 23 carbon atoms, preferably 11 to 21 carbon atoms and from 1 to 3 double bonds; R₆ represents an alkyl or alkenyl group derived from a linear fatty alcohol containing 6 to 24 carbon atoms, preferably from 12 to 20, more preferably from 18 to 20 carbon atoms. The content of component (b1) is in the range from 0.6 to 20.0% wt., more preferably from 1.0 to 12.0% wt. based on the total weight of the composition.

In an embodiment of the present invention, the total weight of the composition refers to the total weight of the sum of component (a), component (b) and component (c), that is ((a)+(b)+(c)).

In an embodiment of the present invention, the component (b1) present in the composition is intentionally added in the esterification step, after the esterification step, in the quaternization step, or after the quaternization step of component (a) and/ or generated in situ in the esterification step.

In another embodiment of the present invention, the component (b1) present in the composition is obtained in the esterification step by the reaction between the fatty acid or mixture of fatty acids and a linear fatty alcohol or mixture of fatty alcohols additionally added into the system.

In another embodiment of the present invention, the component (b1) present in the composition is added to the system after the esterification step has finished, and can act as a solvent for the quaternization step.

In another embodiment of the present invention, the component (b1) present in the composition is added to the component (a) after the quaternization step as an additive.

Yet in another embodiment of the present invention, the component (b1) present in the composition corresponds to the combination of the previously described embodiments.

In another embodiment of the present invention, the component (b1) is represented by formula (II), wherein R₅ represents a fatty acid moiety being a linear alkyl containing 5 to 23 carbon atoms, preferably 11 to 21 carbon atoms, or a linear alkenyl group containing from 5 to 23 carbon atoms, preferably from 11 to 21 carbon atoms and from 1 to 3 double bonds, and wherein the alkyl or alkenyl group proceeds from fatty acids derived from palm oil and/or hydrogenated palm oil, coconut oil, tallow and/or hydrogenated tallow, more preferably from tallow or palm and hydrogenated tallow or palm.

In another embodiment of the present invention, the component (b1) is represented by formula (II), wherein R₆ is derived from a fatty alcohol or mixtures of fatty alcohol represented by formula (VI):

R₁₃-O-(CH₂CH₂O)ₘ-(CH₂CHR₁₄O)ᵥ-H Formula (VI)

wherein R₁₃ represents a linear alkyl or alkenyl group containing 6 to 24 carbon atoms, preferably from 12 to 20 carbon atoms, more preferably from 18 to 20 carbon atoms; R₁₄ represents an alkyl group containing 1 to 4 carbon atoms; m represents a number within the range of 0 to 10, v represents a number within the range of 0 to 10, and the sum of m + v represents the average alkoxylation degree which corresponds to a number from 0 to 20, preferably from 0 to 10, more preferably from 0 to 5.

The group derived from the fatty alcohol can be ethoxylated and/or propoxylated, since m and v can be larger than 0. The order of sequence of the ethylene oxide and propylene oxide groups is not critical for the invention.

In an embodiment of the present invention the fatty alcohol of formula (VI) is not alkoxylated.

In another embodiment of the present invention, the fatty acid moieties in the component (b1) and the component (a) , as defined herein, are derived from the same fatty acid or mixture of fatty acids.

In another embodiment of the present invention, the component (b1), as defined herein, is a fatty acid ester or a mixture of fatty acid esters derived from a C₁₈-C₂₀ fatty alcohol or mixtures of C₁₈-C₂₀ fatty alcohols. Preferably, the component (b1) is a fatty acid ester derived from a fatty alcohol.

In another embodiment of the present invention, the component (b1), as defined herein, is a fatty acid ester or a mixture of fatty acid esters derived from a linear C₁₈-C₂₀ fatty alcohol or mixtures of linear C₁₈-C₂₀ fatty alcohols. Preferably, the component (b1) is a fatty acid ester derived from a linear fatty alcohol.

### Component (b2)

The component (b2) is a fatty acid ester or a mixture of fatty acid esters of formula (III):

R₇-COO-R₈ Formula (III)

wherein R₇ represents a fatty acid moiety being a linear or branched alkyl containing from 5 to 23 carbon atoms, preferably from 11 to 21 carbon atoms, or a linear or branched alkenyl containing 5 to 23 carbon atoms, preferably from 11 to 21 carbon atoms, and 1 to 3 double bonds; R₈ represents an alkyl or alkenyl group derived from a linear or branched fatty alcohol containing from 3 to 24 carbon atoms, preferably from 3 to 10 carbon atoms; with the proviso that at least one of R₇ and R₈ represents a branched alkyl or alkenyl group. The content of (b2) is in the range from 1.0 to 29.0% wt., more preferably from 5 to 20% wt. based on the total weight of the composition.

In an embodiment of the present invention, the component (b2) present in the composition is intentionally added after the esterification step, in the quaternization step, or after the quaternization step of component (a).

In another embodiment of the present invention, the component (b2) present in the composition is added to the system after the esterification step has finished, and can act as a solvent for the quaternization step.

In another embodiment of the present invention, the component (b2) present in the composition is added to the component (a) after the quaternization step as an additive.

Yet in another embodiment of the invention, the component (b2) present in the composition corresponds to the combination of the previously described embodiments.

In an embodiment of the present invention, the component (b2) is represented by formula (III), wherein R₇ represents a fatty acid moiety being a linear or branched alkyl containing 5 to 23 carbon atoms, or a linear or branched alkenyl containing 5 to 23 carbon atoms and from 1 to 3 double bonds; with the proviso that at least one of R₇ and R₈ represents a branched alkyl or alkenyl group. Preferably the alkyl or alkenyl group proceeds from fatty acids derived from palm oil, coconut oil, tallow and hydrogenated tallow, as well as myristic acid, more preferably from fatty acids derived from palm oil or myristic acid.

In an embodiment of the present invention, the component (b2) is represented by formula (III), wherein R₇ represents fatty acid moiety derived from palm oil and hydrogenated palm oil, coconut oil, tallow and hydrogenated tallow, as well as myristic acid or palmitic acid or 2-ethylhexanoic acid, more preferably from fatty acids derived from palm oil or myristic acid.

In another embodiment of the present invention, the component (b2) is represented by formula (III), wherein R₇ represents a C₁₂ alkyl or alkenyl group, a C₁₄ alkyl or alkenyl group, a C₁₆ alkyl or alkenyl group, a alkyl or alkenyl group, or mixtures thereof.

In another embodiment of the present invention, the fatty acid moiety of component (b2) is a branched fatty acid such as isopentanoic acid, 2-ethylhexanoic acid, isononanoic acid, 2,2-dimethylpropanoic acid, neoheptanoic acid, neo-octanoic acid, neononanoic acid, isohexanoic acid, neodecanoic acid, 2-ethylhexanoic acid, 3,5,5-trimethyl, isoheptanoic acid, iso-octanoic acid, isononanoic acid, isostearic acid, isopalmitic acid and isodecanoic acid, pelargonic acid.

In an embodiment of the present invention, the component (b2) is derived from a linear or branched fatty alcohol containing 3 to 24 carbon atoms. Suitable fatty alcohols include linear alcohols such as lauryl alcohol (1-dodecanol), myristil alcohol (1-tetradecanol), cetyl alcohol (1-hexadecanol), stearyl alcohol (1-octadecanol), elaidyl alcohol (9E-octadecen-1-ol), oleyl alcohol (cis-9-octadecen-1-ol), linoleyl alcohol (9Z, 12Z-octadecadien-1-ol), elaidolinoleyl alcohol (9E, 12E-octadecadien-1-ol), behenyl alcohol (1-docosanol), erucyl alcohol (cis-13-docosen-1-ol); and branched fatty alcohols such as 2-ethylhexanol, isopropyl alcohol, 2-methyl-1-pentanol, 2-propyl-1-heptanol, 2-butyl-1-octanol, 2-pentyl-1-nonanol, 2-hexyl-1-decanol, 2-heptyl-1-undecanol, 2-octyl-1-dodecanol, 2-nonyl-1-tridecanol.

In an embodiment of the present invention, the component (b2) is derived from a branched C₃-C₁₀ fatty alcohol such as 2-ethylhexanol or isopropyl alcohol or mixtures thereof.

In an embodiment of the present invention, the fatty acid moieties in the component (b2) and the component (a), as defined herein, are derived from the same fatty acid or mixtures of fatty acids.

In another embodiment of the present invention, the fatty acid moieties in the component (b2) and the component (a), as defined herein, are derived from different fatty acids.

In an embodiment of the present invention, the component (b2), as defined herein, is a fatty acid ester or a mixture of fatty acid esters derived from a C₃-C₁₀ branched fatty alcohol or a mixture of C₃-C₁₀ branched fatty alcohols.

In another embodiment of the present invention, the component (b2) is a fatty acid ester or a mixture of fatty acid esters wherein the fatty acid moiety proceeds from fatty acids derived from palm oil, coconut oil, tallow and hydrogenated tallow, as well as myristic acid, more preferably from fatty acids derived from palm oil or myristic acid; and wherein the fatty acid ester is derived from a branched C₃-C₁₀ branched fatty alcohol or a mixture of C₃-C₁₀ branched fatty alcohols such as 2-ethylhexanol or isopropyl alcohol or mixtures thereof.

In another embodiment of the present invention, the component (b2) is isopropyl myristate, isopropyl palmitate or mixtures thereof.

In an embodiment of the present invention, the weight ratio between component (b1) and component (b2), that is ((b1):(b2)) is from 70:30 to 5:95, preferably from 50:50 to 20:80.

In one embodiment of the present invention, component (b1) and component (b2), as defined herein, are independently added in the esterification step, after the esterification step, in the quaternization step , or after the quaternization step and/or generated in situ in the esterification step or in the quaternization step.

In one embodiment of the present invention, component (b1), as defined herein, is generated in situ in the esterification step or in the quaternization step, and component (b2), as defined herein, is intentionally added after the esterification step, in the quaternization step or after the quaternization step.

In another embodiment of the present invention, the component (b) is a fatty acid ester comprising a component (b1) being a fatty acid ester or a mixture of fatty acid esters derived from a linear C₆ to C₂₄ fatty alcohol or mixtures of C₆ to C₂₄ linear fatty alcohols; and a component (b2) being a fatty acid ester or a mixture of fatty acid esters derived from a C₃-C₁₀ branched fatty alcohol or a mixture of C₃-C₁₀ branched fatty alcohol.

In a preferred embodiment of the present invention, the component (b) consists of components (b1) and (b2), wherein the content of component (b1) is in the range from 0.6 to 20.0% wt and the content of (b2) is in the range from 1.0 to 29.0% wt., based on the total weight of the composition.

### (c): Fatty alcohol

The quaternary ester ammonium composition of the present invention comprises a component (c), said component being a fatty alcohol or a mixture of fatty alcohols of formula (IV),

R₉-O-(CH₂CH₂O)ₗ-(CH₂CHR₁₀O)ₖ-H Formula (IV)

wherein R₉ represents an alkyl or alkenyl group containing 6 to 24 carbon atoms, preferably from 12 to 20 carbon atoms, more preferably from 18 to 20 carbon atoms, R₁₀ represents an alkyl group containing 1 to 4 carbon atoms; 1 represents a number within the range of 0 to 10, k represents a number within the range of 0 to 10, and the sum of l+k represents the average alkoxylation degree which corresponds to a number from 0 to 20, preferably from 0 to 10, more preferably from 0 to 5. The component (c) content is in the range from 0.4 to 9.0% wt. based on the total weight of composition, more preferably from 1.0 to 7.0% wt. based on the total weight of the composition.

The component (c), as defined herein, of the composition is intentionally added in the esterification step, after the esterification step, in the quaternization step, or after the quaternization step and/or accounts for an unreacted material.

In one embodiment of the present invention, the component (c), as defined herein, present in the composition corresponds to a free or unreacted fatty alcohol or mixtures of fatty alcohol that has not reacted with a fatty acid in the esterification step to form a fatty acid fatty alcohol ester.

In another embodiment, the component (c) present in the composition, as defined herein, corresponds to a fatty alcohol or mixture of fatty alcohols added to the esterification product as a quaternizing solvent.

In another embodiment, the component (c), as defined herein, present in the composition is added to the component (a) after the quaternization step as an additive.

Yet in another embodiment of the present invention, the component (c), as defined herein, present in the composition corresponds to the combination of the previously described embodiments.

Suitable fatty alcohol is a C₆ to C₂₄ alcohol or mixture of C₆ to C₂₄ alcohol, preferably from C₁₂ to C₂₀ carbon atoms, more preferably from 18 to 20 carbon atoms.

In an embodiment of the present invention, the fatty alcohol is a linear fatty alcohol. Suitable linear fatty alcohols include capryl alcohol (1-octanol), pelargonic alcohol (1-nonanol), capric alcohol (1-decanol), lauryl alcohol (1-dodecanol), lauryl alcohol (1-dodecanol), myristil alcohol (1-tetradecanol), cetyl alcohol (1-hexadecanol), stearyl alcohol (1-octadecanol), elaidyl alcohol (9E-octadecen-1-ol), oleyl alcohol (cis-9-octadecen-1-ol), linoleyl alcohol (9Z, 12Z-octadecadien-1-ol), elaidolinoleyl alcohol (9E, 12E-octadecadien-1-ol), linolenyl alcohol (9Z, 12Z, 15Z-octadecatrien-1-ol), elaidolinolenyl alcohol (9E, 12E, 15-E-octadecatrien-1-ol), arachidyl alcohol (1-eicosanol), behenyl alcohol (1-docosanol), and erucyl alcohol (cis-13-docosen-1-ol) and mixtures thereof.

In an embodiment of the invention, the weight ratio among component (b1), component (c) and component (b2), that is (b1):(c):(b2), is from 67.5:7.5:25 to 3:2:95, preferably from 35:15:50 to 18:7:75.

In another embodiment of the invention, the weight ratio of the sum of component (b1) and (c) to component (b2), that is ((b1)+(c)):(b2), is from 75:25 to 5:95, preferably from 50:50 to 25:75.

In another embodiment of the invention, the weight ratio between component (b1) and component (c), that is (b1):(c), is from 60:40 to 90:10, preferably from 65:35 to 80:20.

In another embodiment of the invention, the weight ratio between component (c) and component (b2), that is (c):(b2), is from 70:30 to 1:99, preferably from 50:50 to 10:90.

In another embodiment of the invention, the weight ratio of the component (a) to the sum of component (b) and component (c), that is (a): ((b)+(c)), is preferably from 95:5 to 70:30, more preferably from 90:10 to 75:25. In this embodiment, the component (b) preferably consists of components (b1) and (b2).

The composition according to the present invention may contain further components.

### (d): Solvent

The present invention may further comprise a component (d), said component being a solvent. In a preferred embodiment, the component (d) content is 0% wt. or more and lower than 12% wt., preferably 0% wt. or more and lower than 9% wt., more preferably 0% wt. or more and lower than 6% wt. based on the total weight of the composition.

Solvents useful as component (d) in the present technology include flammable liquids of flash point equal to or lower than 40°C selected from the following list: methanol, ethanol, 1-propanol, 2-propanol, 1-butanol, 2-butanol, hexane, heptane, and combinations thereof. Preferably, the solvent is ethanol or 2-propanol and most preferably 2-propanol.

Other solvents suitable for use as component (d) in the present invention include ethylene glycol, trimethylene glycol, tetramethylene glycol, pentamethylene glycol, hexamethylene glycol, diethylene glycol, triethylene glycol, propylene glycol, dipropylene glycol and C₁-C₄ alkyl monoethers of ethylene glycol, propylene glycol, and dipropylene glycol, sorbitol, alkane diols such as 1,2-propanediol, 1,3-propanediol, 2,3-butanediol, 1,4-butanediol, 1,3-butanediol, 1,5-pentanediol, and 1,6 hexanediol; phenylethyl alcohol, 2-methyl-1,3-propanediol, hexylene glycol, sorbitol, polyethylene glycols, 1,2-hexanediol, 1,2-pentanediol, 1,2-butanediol, 1,4-cyclohexanedimethanol, pinacol, 2,4-dimethyl- 2,4-pentanediol, 2,2,4-trimethyl-1,3-pentanediol (and ethoxylates), 2-ethyl-1,3-hexanediol, phenoxyethanol (and ethoxylates), glycol ethers, butyl carbitol, dipropylene glycol n-butyl ether, or combinations thereof.

### Further preferred embodiments of the quaternary ester ammonium composition

Preferably, the composition of the present invention comprises:
- a component (a), said component comprising at least one or more quaternary mono-, di- or tri-ester ammonium compounds of formula (I1), (I2), (I3), wherein the content of quaternary ester ammonium compounds in the composition is in the range from 70 to 95% wt., more preferably from 75 to 90% wt. based on the total weight of the composition;
- a component (b), said component being a fatty acid ester comprising at least a component (b1) being a fatty acid ester of formula (II), as defined herein, wherein the component (b1) content is in the range from 0.6 to 20.0% wt. based on the total weight of the composition, more preferably from 1.0 to 12.0% wt., and at least a component (b2) being a fatty acid ester of formula (III), as defined herein, wherein the component (b2) content is in the range from 1.0 to 29.0% wt., more preferably from 5.0 to 20.0% wt. based on the total weight of the composition.
- a component (c), said component being a C₆-C₂₄ fatty alcohol, preferably a C₁₂-C₂₀ fatty alcohol, wherein the component (c) content is in the range from 0.4 to 9.0% wt. based on the total weight of the composition, more preferably from 1.0 to 7.0 % wt. based on the total weight of the composition.

In one preferred embodiment of the present invention, the component (a) comprises or consists of at least one quaternary mono-ester ammonium compound of formula (I1), at least one quaternary di-ester ammonium compound of formula (I2), and at least one quaternary tri-ester ammonium compound of formula (I3), wherein m=n=p=2; R₁-C(O)- is a linear acyl group wherein R₁ is a linear alkyl or alkenyl containing from 11 to 21 carbon atoms, preferably derived from (hydrogenated or non-hydrogenated) tallow fatty acid or palm fatty acid; R₂ and R₃ each represent -OH, q is 0 (i.e. the compound is not alkoxylated); X₁, is a methyl group; and A⁻ is selected from a halide, phosphate or alkylsulphate, preferably alkylsulphate. Such a compound may be produced by esterifying tallow fatty acid and triethanolamine, wherein the molar ratio of tallow fatty acid to alkanolamine is 1.4-2.5, preferably 1.5-2.2, and subsequently methylating the esteramine obtained thereby, wherein the degree of quaternization is from 25 to 95 mol-%.

In another preferred embodiment of the present invention, the component (b1) is a fatty acid ester or mixture of fatty acid esters of formula (II), as defined herein, wherein the fatty acid source is preferably palm oil, coconut oil, tallow and hydrogenated tallow, more preferably palm oil and/or tallow and hydrogenated tallow, and the fatty alcohol source is a linear fatty alcohol containing from 6 to 24 carbon atoms, wherein the component (b1) content is in the range from 0.6 to 20.0% wt. based on the total weight of the composition.

In another preferred embodiment of the present invention, the component (b2) is a fatty acid ester or mixture of fatty acid esters of formula (III), as defined herein, wherein the fatty acid source is preferably palm oil or myristic acid, and the fatty alcohol source is C₃-C₁₀ branched fatty alcohol or a mixture of C₃-C₁₀ branched fatty alcohols, preferably 2-ethylhexanol or isopropyl alcohol, wherein the component (b2) content is in the range from 1.0 to 29.0% wt based on the total weight of the composition.

In another preferred embodiment of the present invention, the component (c) is a C₆ to C₂₄ linear fatty alcohol wherein the component (c) content is in the range from 0.4 to 9.0% wt. based on the total weight of the composition.

In another preferred embodiment of the present invention, the composition further comprises a component (d), said component being a solvent, wherein the solvent content is higher than 0% and lower than 8% wt., preferably lower than 6% wt. based on the total weight of the composition.

In another preferred embodiment of the present invention, the component (d) is chosen from ethanol, 1-propanol and 2-propanol. The component (d) is preferably ethanol or 2-propanol and most preferably 2-propanol.

In the present invention, the composition comprises, in the indicated amounts expressed as percentage by weight with respect to the total weight of the composition:
- at least one or more quaternary mono-, di- or tri-ester ammonium compounds of the component (a),
- 0.6 to 20.0% of the component (b1),
- 1.0 to 29.0% of the component (b2),
- 0.4 to 9.0% of the component (c).

In another preferred embodiment of the invention, the composition comprises, in the indicated amounts expressed as percentage by weight with respect to the total weight of the composition:
- at least one or more quaternary mono-, di- or tri-ester ammonium compounds of the component (a),
- 0.6 to 20.0% of the component (b1),
- 1.0 to 29% of the component (b2),
- 0.4 to 9.0% of the component (c),
- 0 to 8% of the component (d).

In any of the above embodiments or preferred embodiments, the sum of amounts of components (a), (b1), (b2) and (c), together with inevitable impurities, preferably adds up to 100% by weight with respect to the total weight of the composition.

In another embodiment of the invention, if (d) is present, the content of (d) is based on the sum of (a), (b1), (b2), (c) and (d), that is (d)/(a+b1+b2+c+d).

Preferably, the weight ratio between component (b1) and component (b2), that is ((b1):(b2)) is from 70:30 to 5:95, more preferably from 50:50 to 20:80. The weight ratio among component (b1), component (c) and component (b2), that is (b1):(c):(b2) is preferably from 67.5:7.5:25 to 3:2:95, more preferably from 35:15:50 to 18:7:75; the weight ratio between the sum of component (b1) and (c), and component (b2), that is ((b1)+(c)):(b2) is preferably from 75:25 to 5:95, more preferably from 50:50 to 25:75; the weight ratio between component (b1) and component (c), that is (b1):(c) is preferably from 60:40 to 90:10, more preferably from 65:35 to 80:20; the weight ratio between component (c) and component (b2), that is (c):(b2) is preferably from 70:30 to 1:99, more preferably from 50:50 to 10:90; and the weight ratio between the component (a) and the sum of component (b) and component (c), that is (a):((b)+(c)) is preferably from 95:5 to 70:30, more preferably from 90:10 to 75: 25.

In an embodiment of the present invention, the composition contains from 70 to 95% wt., more preferably from 75 to 90% wt. of quaternary ester ammonium compounds based on the total weight of the composition.

In an embodiment of the present invention, the composition preferably contains water in an amount of 0% wt. or more and lower than 2% wt., more preferably 0% wt. or more and lower than 1% wt. based on the total weight of the composition; and is most preferably essentially water-free.

The composition can be used to soften fabrics by treating the fabric with the composition. The composition can also be used to condition hair by treating hair with the composition.

### PROCESS TO OBTAIN THE QUATERNARY ESTER AMMONIUM COMPOSITION

A process to obtain a composition according to the present invention comprises:
i) an esterification step, wherein a fatty acid, a methyl ester or a triglyceride thereof is reacted with an alkanolamine to obtain a mixture containing an esteramine; and
ii) a quaternization step, wherein the mixture obtained after the esterification step is reacted with an alkylating agent.

Preferably, a C₆-C₂₄ linear fatty alcohol is added in the esterification step, after the esterification step, in the quaternization step or after the quaternization step. The term "added in the esterification/quaternization step" as used herein refers to addition of the respective component to the esterification/quaternization reaction mixture either prior to or in course of the esterification/quaternization reaction.

The process to obtain a composition according to the present invention is characterized in that the component (b1) present in the composition is intentionally added in the esterification step, after the esterification step, in the quaternization step or after the quaternization step and/or generated in situ in the esterification step or in the quaternization step; and component (b2) present in the composition is intentionally added after the esterification step, in the quaternization step or after the quaternization step.

In an embodiment of the invention, the process to obtain the composition is characterized in that the component (b1) present in the composition is generated in situ in the esterification step and component (b2) is intentionally added after the esterification step, in the quaternization step or after the quaternization step.

In another embodiment of the present invention, the process to obtain the composition is characterized in that component (b1) and component (b2) are independently intentionally added in the esterification step, after the esterification step, in the quaternization step or after the quaternization step as additives.

The process to obtain a composition according to the present invention is also characterized in that the component (c) present in the composition is intentionally added in the esterification step, after the esterification step, in the quaternization step or after the quaternization step and/or corresponds to unreacted material.

### FABRIC SOFTENER COMPOSITION

Another object of the present invention is a fabric softener composition comprising a quaternary ester ammonium composition that comprises a component (a), a component (b) comprising at least a component (b1) and a component (b2), a component (c) and optionally a component (d) as defined above, further comprising at least water. The fabric softener composition preferably comprises the quaternary ester ammonium composition in an amount from 1 to 30% wt., more preferably from 1.5 to 25% wt., most preferably from 2 to 20% wt. based on the total weight of the fabric softener composition.

In one embodiment of the present invention, the fabric softener composition further comprises optional components. In referring to the optional components, without this having to be regarded as an exhaustive description of all possibilities, which, on the other hand, are well known to the person skilled in the art, the following may be mentioned:
a) other products that enhance the performance of the softener compositions, such as silicones, amine oxides, anionic surfactants, such as lauryl ether sulphate or lauryl sulphate, amphoteric surfactants, such as cocoamidopropyl betaine or alkyl betaines, sulphosuccinates, polyglucoside derivatives, etc.
b) stabilising products, such as salts of amines having a short chain, which are quaternised or non-quaternised, for example of triethanolamine, N-methyldiethanolamine, etc., and also non-ionic surfactants, such as ethoxylated fatty alcohols, ethoxylated fatty amines.
c) products that improve viscosity control, such as inorganic salts, for example, calcium chloride, magnesium chloride, calcium sulphate, sodium chloride, etc.; products which can be used to reduce viscosity in concentrated compositions, such as compounds of the glycol type, for example, ethylene glycol, dipropylene glycol, polyglycols, etc.; thickening agents for diluted compositions, such as polymers, suitable polymers are water soluble or dispersible, preferably the polymers are cationic. Suitable cationic polymeric materials include cationic guar polymers, cationic cellulose derivatives, cationic potato starch, cationic polyacrylamides. Specially suitable are cross-linked water swellable cationic polymers. Those described polymers may also act as deposition aids.
d) components for adjusting the pH, which is from 2.0 to 6.0, preferably from 2.5 to 4.0, such as any type of inorganic and/or organic acid, for example hydrochloric, sulphuric, phosphoric, lactic acid, citric acid etc.
e) agents that improve soil release, such as the known polymers or copolymers based on terephthalates.
f) preservatives, such as bactericides, for example, 1,2-benzisothiazolin-3-one, 5-chloro-2-methyl-4-isothiazolin-3-one and 2-methyl-4-isothiazolin-3-one, or their combinations, 2-bromo-2-nitropropane-1,3-diol, etc.
g) other products such as antioxidants, coloring agents, perfumes, germicides, fungicides, anti-corrosive agents, anti-crease agents, opacifiers, optical brighteners, pearl luster agents, etc.

In an embodiment of the present invention, the fabric softener composition preferably comprises a perfume, more preferably a neat perfume or a perfume microcapsule. The perfume content is preferably lower than 5% wt., preferably lower than 3% wt., more preferably lower than 2% wt. based on the total weight of the fabric softener composition.

In a particularly preferred embodiment of the present invention, the fabric softener composition comprises:
a) from 0 to 5% of non-ionic surfactant(s), preferably from 0 to 3%, more preferably from 0 to 1.5%, and/or
b) from 0 to 2% of electrolyte concentration aid(s), preferably from 0.01 to 1%, more preferably from 0.02 to 0.5%; and/or
c) from 0.01 to 3% of thickening polymer(s), preferably from 0.02 to 1%, more preferably from 0.05 to 0.5%; and/or
d) from 0.01 to 5% of perfume(s), alternatively from 0.1 to 4% or from 0.2 to 4% of a neat perfume and optionally from 0.01 to 3%, preferably from 0.1 to 2%, more preferably from 0.3 to 1%, of a perfume microcapsule,
wherein the amounts are expressed as percentage by weight with respect to the total weight of the fabric softener composition.

Suitable non-ionic surfactants include ethoxylated fatty alcohols, ethoxylated fatty amines, hydrophilic and lipophilic non-ionic surfactants, as described in detail further below.Suitable electrolyte concentration aids include salts such as alkali metal, alkaline earth metal and ammonium halides, sulphates, nitrates, carbonates and bicarbonates.

Suitable thickening polymers include water soluble or dispersible, preferably cationic polymers, as described above. Suitable cationic polymeric materials include cationic guar polymers, cationic cellulose derivatives, cationic potato starch, cationic polyacrylamides. Especially suitable are cross-linked water swellable cationic polymers, such as copolymers of acrylamide with dimethylaminoethyl methacrylate quaternized with methyl chloride, preferably copolymers of about 20 mol-% acrylamide and about mol-80 % dimethyl amino ethyl methacrylate methyl chloride cross-linked with from 450 to 600 ppm of methylene bisacrylamide (such as FLOSOFT 222 manufactured by SNF). Suitable perfumes include perfume oils (including mixtures of natural and synthetic fragrances), as described in detail further below.

### PREPARATION OF FABRIC SOFTENER COMPOSITION

The fabric softener composition of the present invention can be obtained following a conventional process of mixing the different components, well known by any skilled person. For example, the different components can be mixed in the molten state, added to the water and stirred to obtain a homogeneous dispersion and then cooled down. In a preferred process of obtention, non-ionics, electrolytes and/or polymers, if present in the composition, are added to the water previous to the dispersion of the composition, or once the composition is dispersed in water. Perfume can be added once the quaternary ester ammonium composition is dispersed in water and the blend is cooled down.

### METHOD FOR CONDITIONING TEXTILES

The fabric softener composition according to the invention can be used in both a so-called non-rinse and a so-called rinse process, where a fabric softener composition as defined above is first diluted in an aqueous rinse bath solution. Subsequently, the laundered fabrics which have been washed with a detergent liquor and optionally rinsed in one or more inefficient rinse steps ("inefficient" in the sense that residual detergent and/or soil may be carried over with the fabrics) are placed in the rinse solution with the diluted composition. Of course, the fabric softener composition may also be incorporated into the aqueous bath once the fabrics have been immersed therein. Following that step, agitation is applied to the fabrics in the rinse bath solution causing the suds to collapse, and residual soils and surfactant are to be removed. The fabrics can then be optionally wrung before drying.

The non-rinse/rinse process may be performed manually in a basin or bucket, in a non-automated washing machine, or in an automated washing machine. When hand washing is performed, the laundered fabrics are removed from the detergent liquor and wrung out. The fabric softener of the present invention is then added to fresh water and the fabrics are then, directly in case of the non-rinse process or after one or more optional inefficient rinse steps in case of the rinse process, rinsed in the water containing the composition according to the conventional rinsing habit. The fabrics are then dried using conventional means.

The fabric softener composition can be used to soften fabrics by treating the fabric with the composition. This can be done during the non-rinse and rinse process using a liquid fabric softener.

In an embodiment of the invention, the method for conditioning textiles of fabrics comprises the steps of contacting one or more fabric articles with the fabric softener composition of the invention at one or more points during the laundering process, and allowing the fabric articles to dry or mechanically tumble-drying them.

The use of the fabric conditioner composition of the invention for the conditioning treatment of textiles is another embodiment of the invention.

### HAIR CONDITIONING COMPOSITION

Another object of the present invention is a hair conditioner composition comprising a quaternary ester ammonium composition that comprises a component (a), a component (b) comprising at least a component (b1) and a component (b2), a component (c) and optionally a component (d), as defined above, further comprising at least water, wherein the composition comprises, in the indicated amounts expressed as percentage by weight with respect to the total weight of the composition:
0.6 to 20.0% of component (b1),
1.0 to 29.0% of component (b2), and
0.4 to 9.0% of component (c).

The hair conditioner composition preferably comprises the quaternary ester ammonium composition in an amount from 1 to 30% wt. based on the total weight of the hair conditioning composition, more preferably from 1.5 to 25% wt., most preferably from 2 to 20% wt.

In one embodiment of the present invention, the hair conditioner composition further comprises optional components. The hair conditioner composition may also comprise oil components, silicone compounds, powders, amphoteric surfactants, non-ionic surfactants, polymers, metal ion sequestering agents, UV protection factors, vitamins, antioxidants, antioxidant aids, perfume oils, germ inhibitors and the like as further auxiliaries and additives.

Examples of oils include liquid oils, solid oils, waxes, hydrocarbon oils and synthetic ester oils. Suitable oil components are, for example, Guerbet alcohols based on fatty alcohols containing 6 to 22 and preferably 8 to 10 carbon atoms, esters of linear C₆-C₂₂ fatty acids with linear C₆-C₂₂ fatty alcohols, esters of branched C₆-C₂₂ carboxylic acids with linear C₆-C₂₂ fatty alcohols such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isopropyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl cleats, erucyl behenate and erucyl erucate. Also suitable are esters of linear C₆-C₂₂ fatty acids with branched alcohols, more particularly 2-ethyl hexanol, esters of hydroxycarboxylic acids with linear or branched C₆-C₂₂ fatty alcohols, esters of linear and/or branched fatty acids with polyhydric alcohols for example propylene glycol, dimer diol or trimer triol) and/or Guerbet alcohols, triglycerides based on C₆-C₁₀ fatty acids, liquid mono-/di-/triglyceride mixtures based on C₆-C₁₈ fatty acids, esters of C₆-C₂₂ fatty alcohols and/or Guerbet alcohols with aromatic carboxylic acids, more particularly benzoic acid, esters of C₆-C₁₂ dicarboxylic acids with linear or branched alcohols containing 1 to 22 carbon atoms or polyols containing 2 to 10 carbon atoms and 2 to 6 hydroxyl groups, vegetable natural oils such as avocado oil, almond oil, hazelnut oil, babassu palm oil, borage oil, peanut oil, jojoba oil, canola oil, hemp oil, soybean oil, milk thistle oil, safflower oil, chufa oil, coconut oil, rapeseed oil, black cumin oil, wheat germ oil, sunflower oil, linseed oil, macadamia nut oil, corn oil, walnut oil, olive oil, branched primary alcohols, substituted cyclohexanes, linear and branched C₆-C₂₂ fatty alcohol carbonates, Guerbet carbonates, esters of benzoic acid with linear and/or branched C6-C22 alcohols, linear or branched, symmetrical or non-symmetrical dialkyl ethers containing 6 to 22 carbon atoms per alkyl group, ring opening products of epoxidized fatty acid esters with polyols, silicone oils and/or aliphatic or naphthenic hydrocarbons, for example dialkyl cyclohexanes.

Examples of waxes include natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes, microwaxes; chemically modified waxes (hard waxes) such as for example, montan ester waxes, sasol waxes, hydrogenated jojoba waxes and synthetic waxes such as, for example, polyalkylene waxes and polyethylene glycol waxes.

Examples of hydrocarbon oils include liquid paraffin, squalane, pristane, paraffin, ceresin, squalene, petrolatum, and microcrystalline wax.

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Preferred silicone compounds are hydrophobic silicone oils, which are silicone oils which are soluble in paraffinic oil at 25°C. Hydrophobic silicone oils to be used according to the present invention include both volatile and non-volatile silicone oils.

Specific examples include a cyclic methyl siloxane having the formula {(CH₃)₂SiO}ₓ in which x is 3-6, or short chain linear methyl siloxanes having the formula ((CH3)₂SiO{(CH₃)₂SiO}_{y}Si(CH₃)₃ in which y is 0-5.

Some suitable cyclic methyl siloxanes are hexamethylcyclotrisiloxanes (D3), a solid with a boiling point of 134°C and the formula {(Me₂)SiO}₃; octamethylcyclotetrasiloxane (D4) with a boiling point of 176°C, a viscosity of 2.3 mm²/s, and the formula {(Me₂)SiO}₄ decamethylcyclopentasiloxane (D5) (cyclomethicone) with a boiling point of 210°C, a viscosity of 3.87 mm²/s, and the formula {(Me₂) SiO}₅; and dodecamethylcyclohexasiloxane (DE) with a boiling point of 245°C, a viscosity of 6.62 mm²/s and the formula {(Me₂)SiO}₆.

Some suitable short linear methyl siloxane are hexamethyldisiloxane (MM) with a boiling point of 100°C, viscosity of 0-65 mm²/s, and formula Me₃SiOMe₃; octamethyltrisiloxane (MDM) with a boiling point of 152°C., viscosity of 1.04 mm²/s, and formula Me₃SiOMe₂SiOSiMe₃; decamethyltetrasiloxane (MD2M) with a boiling point of 194°C, viscosity of 1.53 mm²/s, and formula Me₃SiO(MeSiO)₂SiMe₃; dodecamethylpentasiloxane (MD3M) with a boiling point of 229°C, viscosity of 2.06 mm²/s, and formula Me₃SiO(Me₂SiO)₃SiMe₃ tetradecamethylhexasiloxane (MD4M) with a boiling point of 245°C, viscosity of 2.63 mm²/s, and formula Me₃SiO(Me₂SiO)₄SiMe₃; and hexadecamethylheptasiloxane (MD5M) with a boiling point of 270°C, viscosity of 3.24 mm²/s, and formula Me₃SiO(Me₂SiO)₅SiMe₃.

Furthermore, long chain linear siloxanes such as phenyl trimethicone, bis(phenylpropyl)dimethicone, dimethicone, and dimethiconol are also included.

Examples of powders include inorganic powders such as talc, kaolin, mica, sericite, muscovite, phlogopite, synthetic mica, lepidolite, biotite, vermiculite, bentonite, hectorite, laponite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate (calcined gypsum), calcium phosphate, fluorine apatite, hydroxyapatite, ceramic powder, metallic soap (e.g., zinc myristate, calcium palimitate, and aluminum stearate), and boron nitride; organic powders such as polyamide resin powder (nylon powder), polyethylene powder, polymethylmethacrylate powder, polystyrene powder, styrene-acrylic acid copolymer resin powder, benzoguanamine resin powder, poly(tetrafluroethylene) powder, and cellulose powder; inorganic white pigments such as titanium dioxide and zinc oxide; inorganic red pigments such as iron oxide (red iron oxide) and iron titanate; inorganic brown pigments such as [gamma]-iron oxide; inorganic yellow pigments such as yellow iron oxide and ocher; inorganic black pigments such as black iron oxide and lower order titanium oxide; inorganic purple pigments such as mango violet and cobalt violet; inorganic green pigments such as chrome oxide, chrome hydroxide, and cobalt titanate; inorganic blue pigments such as ultramarine and Prussian blue; pearl pigments such as titanium oxide coated mica, titanium oxide coated bismuth oxychloride, titanium oxide coated talc, colored titanium oxide coated mica, bismuth oxychloride, and fish scale flakes; metal powder pigments such as aluminum powder and copper powder; organic pigments such as zirconium, barium, or aluminum lake (e.g., organic pigments such as Red No.201, Red No.202, Red No.204, Red No.205, Red No.220, Red No.226, Red No.228, Red No.405, Orange No.203, Orange No.204, Yellow No.205, Yellow No.401, and Blue No.404,or Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1); and natural colors such as chlorophyll and [beta]-carotene.

Examples of amphoteric surfactants include imidazoline type amphoteric surfactants such as sodium 2-undecyl-N,N,N-(hydroxyethylcarboxymethyl)-2-imidazoline and 2-cocoyl-2-imidazolinium hydroxide-1-carboxyethyloxy)-2-sodium salt; betaine type surfactants such as 2-heptadecyl-N-carboxymethyl-N-hydroxyethylimidazolinium betaine, lauryldimethyl aminoacetate betaine, alkyl betaine, amidobetaine, and sulfobetaine.

Examples of lipophilic non-ionic surfactants suitable for the hair conditioning or fabric softening compositions described herein include sorbitan fatty acid esters (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerol or polyglycerol fatty acid esters (such as glycerol mono-cotton seed oil fatty acid ester, glycerol monoerucate, glycerol sesquioleate, glycerol monostearate, glycerol [alpha], [alpha]'-oleate pyroglutamate, and glycerol monostearate malate); propylene glycol fatty acid esters (such as propylene glycol monostearate); hardened castor oil derivatives; and glycerol alkyl ethers.

Examples of hydrophilic non-ionic surfactants suitable for the hair conditioning or fabric softening compositions described herein include POE-sorbitan fatty acid esters (such as POE-sorbitan monooleate, POE-sorbitan monostearate, and POE-sorbitan tetraoleate); POE sorbitol fatty acid esters (such as POE-sorbitol monolaurate, POE-sorbitol monooleate, POE-sorbitol pentaoleate, and POE-sorbitol monostearate); POE-glycerol fatty acid esters (such as POE-monooleates, POE-glycerol monostearate, POE-glycerol monoisostearate, and POE-glycerol triisostearate); POE-fatty acid esters (such as POE-distearate, POE-monodioleate, and ethylene glycol distearate); POE-alkyl ethers (such as POE-lauryl ether, POE-oleyl ether, POE-stearyl ether, POE-behenyl ether, POE-2-octyldodecyl ether, and POE-cholestanol ether); Pluronic type surfactants (such as Pluronic); POE/POP-alkyl ethers (such as POE/POP cetyl ether, POE/POP 2-decyltetradecyl ether, POE/POP monobutyl ether, POE/POP hydrogenated lanolin, and POE/POP glycerol ether); tetra POE/tetra POP-ethylenediamine condensates (such as Tetronic); POE-castor oil or hardened castor oil derivatives (such as POE-castor oil, POE-hardened castor oil, POE-hardened castor oil monoisostearate, POE- hardened castor oil triisostearate, POE-hardened castor oil monopyroglutamate monoisostearate diester, and POE-hardened castor oil maleate); POE-beeswax lanolin derivatives (such as POE-sorbitol beeswax); alkanolamides (such as coconut oil fatty acid diethanolamide, lauric acid monoethanolamide, and fatty acid isopropanolamide); POE-propylene glycol fatty acid esters; POE-alkylamines; POE-fatty acid amides; sucrose fatty acid esters; alkylethoxydimethylamine oxides; and trioleyl phosphate.

Suitable cationic polymers are, for example, cationic cellulose derivatives such as, for example, the quaternized hydroxyethyl cellulose obtainable from Amerchol under the name of Polymer JR 400, cationic starch, copolymers of diallyl ammonium salts and acrylamides, quaternized vinyl pyrrolidone/vinyl imidazole polymers such as, for example, Luviquat (BASF), condensation products of polyglycols and amines, quaternized collagen polypeptides such as, for example, Lauryidimonium Hydroxypropyl Hydrolyzed Collagen, quaternized wheat polypeptides, polyethyleneimine, cationic silicone polymers such as, for example, Amodimethicone, copolymers of adipic acid and dimethylamino-hydroxypropyl diethylenetriamine (Cartaretine, Sandoz), copolymers of acrylic acid with dimethyl diallyl ammonium chloride, polyquaternium type polymers, polyaminopolyamides and crosslinked water-soluble polymers thereof, cationic chitin derivatives such as, for example, quaternized chitosan, optionally in micro-crystalline distribution, condensation products of dihaloalkyls, for example dibromobutane, with bis-dialkylamines, for example bis-dimethylamino-1,3-propane, cationic guar gum such as, for example, Jaguar CBS, Jaguar C-17, Jaguar C-16 of Celanese, quaternized ammonium salt polymers such as, for example, Mirapol A-15, Mirapol AD-1, Mirapol AZ-1 of Mirapol.

Suitable anionic, zwitterionic, amphoteric and non-ionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl, acetate copolymers, vinyl pyrrolidone/dimethylaminoethyl methacrylate/vinyl caprolactam terpolymers and optionally derivatized cellulose ethers and silicones. Examples of UV protection factors include organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances: 3-benzylidene camphor or 3-benzylidene norcamphor and derivatives thereof, for example 3-(4-methylbenzylidene)-camphor; 4-aminobenzoic acid derivatives, preferably 4-(dimethylamino)-benzoic acid-2-ethylhexyl ester, 4-(dimethylamino)-benzoic acid-2-octyl ester and 4-(dimethylamino)-benzoic acid amylester; esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene); esters of salicylic acid, preferably salicylic acid-2-ethylhexyl ester, salicylic acid-4-isopropylbenzyl ester, salicylic acid homomethyl ester;derivatives of benzophenone, preferably 2-hydroxy-4-methoxybenzo-phenone, 2-hydroxy-4-methoxy-4'-methylbenzophenone, 2,2'-dihydroxy-4-methoxybenzophenone; esters of benzalmalonic acid, preferably 4-methoxybenzalmalonic acid di-2-ethylhexyl ester; triazine derivatives such as, for example, 2,4,6-trianilino-(p-carbo-2'-ethyl-1'-hexyloxy)-1,3,5-triazine and Octyl Triazone; propane-1,3-diones such as, for example, 1-(4-tert.butylphenyl)-3-(4T-methoxyphenyl)-propane-1,3-dione; 2-phenylbenzimidazole-5-sulfonic acid and alkali metal, alkaline earth metal, ammonium, alkylammonium, alkanolammonium and glucammonium salts thereof; sulfonic acid derivatives of benzophenones, preferably 2-hydroxy-4-methoxybenzophenone-5-sulfonic acid and salts thereof; sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example 1-(4'-tert.butylphenyl)-3-(4'-methoxyphenyl)-propane-1,3-dione, 4-tert-butyl-4'-methoxydibenzoyl methane (Parsol 1789) or 1-phenyl-3-(4'-isopropylphenyl)-propane-1,3-dione.

The UV-A and UV-B filters may of course also be used in the form of mixtures. Besides the soluble substances mentioned, insoluble pigments, i.e. finely dispersed metal oxides or salts, may also be used for this purpose. Examples of suitable metal oxides are, in particular, zinc oxide and titanium dioxide and also oxides of iron, zirconium, silicon, manganese, aluminum and cerium and mixtures thereof. Silicates (talcum), barium sulfate and zinc stearate may be used as salts. The oxides and salts are used in the form of the pigments for skin-care and skin-protecting emulsions and decorative cosmetics. The particles should have an average diameter of less than 100 nm, preferably from 5 to 50 nm and more preferably from 15 to 30 nm. They may be spherical in shape although ellipsoidal particles or other non-spherical particles may also be used. The pigments may also be surface-treated, i.e. hydrophilicized or hydrophobicized. Typical examples are coated titanium dioxides such as, for example, Titandioxid T 805 (Degussa) or Eusolex T2000 (Merck). Suitable hydrophobic coating materials are, above all, silicones and especially trialkoxyoctyl silanes or simethicones. So-called micro- or nanopigments are preferably used in sun protection products. Micronized zinc oxide is preferably used.

Besides the two above-mentioned groups of primary protection factors, secondary protection factors of the antioxidant type may also be used. Secondary sun protection factors of the antioxidant type interrupt the photochemical reaction chain which is initiated when UV rays penetrate into the skin. Typical examples of suitable antioxidants are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example [alpha]-carotene, [beta]-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, [gamma]-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages (also (metal) chelators (for example ([alpha]-hydroxyfatty acids, palmitic acid, phytic acid, lactoferrine), [alpha]-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example [gamma]-linolenic acid, linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, ([alpha]-glycosyl rutin, ferulic acid, furfurylidene glucitol, carnosine, butyl hydroxytoluene, butyl hydroxyanisole, nordihydroguaiac resin acid, nordihydroguaiaretic acid, trihydroxy-butyrophenone, uric acid and derivatives thereof, mannose and derivatives thereof, Superoxid-Dismutase, zinc and derivatives thereof (for example ZnO, ZnSO4), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Examples of metal ion sequestering agents include 1-hydroxyethane-1,1-diphosphonic acid, 1-hydroxyethane-1,1-diphosphonic acid 4Na salt, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of vitamins include vitamins A, B1, B2, B6, C, and E and the derivatives thereof; pantothenic acid and the derivatives thereof; and biotin.

Examples of antioxidants include tocopherols, dibutylhydroxytoluene, butylhydroxyanisole, and gallic acid esters. Examples of antioxidant aids include phosphoric acid, citric acid, ascorbic acid, maleic acid, malonic acid, succinic acid, lactic acid, fumaric acid, cephalin, hexametaphosphates, phytic acid, and ethylenediaminetetraacetic acid.

Suitable perfume oils are mixtures of natural and synthetic fragrances. Natural fragrances include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamon, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert-butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, filial and bourgeonal. Examples of suitable ketones are the ionones, [alpha]-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable fragrance. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, labolanum oil and lavendin oil. The following are preferably used either individually or in the farm of mixtures: bergamot oil, dihydromyrcenol, lilial, lyral, citronellol, phenylethyl alcohol, [alpha]-nexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, [beta]-damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

Typical examples of germ inhibitors are preservatives which act specifically against gram-positive bacteria such as, for example, 2,4,4'-trichloro-2'-hydroxydiphenyl ether, chlorhexidine (1,6-di-(4-chlorophenyl-biguanido)-hexane) or TCC (3,4,4'-trichlorocarbanilide). Numerous perfumes and essential oils also have antimicrobial properties. Typical examples are the active substances eugenol, menthol and thymol in clove, mint and thyme oil.

In a preferred embodiment of the present invention, the hair conditioner of the present invention comprises at least one additional component selected from the group of emulsifiers, natural vegetable oils and waxes, liquid paraffins and isoparaffins, silicone oils, silicone emulsions, thickeners/viscosity regulators, antioxidants, preservatives, dyes and perfume.

In another preferred embodiment of the present invention, the hair conditioner of the invention comprises:
- a) from 0.5 to 5% of emulsifier(s), preferably from 0.5 to 4% and more preferably from 0.5 to 3%;
- b) from 0.2 to 3% of perfume(s), preferably from 0.2 to 2% and more preferably from 0.2 to 1.5%;
- c) from 0 to 5% of silicone(s), preferably from 0.5 to 3% and more preferably from 0.5 to 2%;
- d) from 0 to 5% of natural oil(s), preferably from 0.5 to 4% and more preferably from 0.5 to 3%;
- e) from 0 to 2% of thickening polymer(s), preferably from 0 to 1% and more preferably from 0 to 0.5%;
wherein the amounts are expressed as percentage by weight with respect to the total weight of the hair conditioner composition. Suitable emulsifiers include anionic emulsifiers, cationic emulsifiers such as distearyldimmonium chloride, stearamidopropyl dimethyl myrystil acetate ammonium chloride; non-ionic emulsifiers such as polysorbates, liquid crystal emulsifiers or polymeric emulsifiers. Suitable perfumes include perfume oils (including mixtures of natural and synthetic fragrances), as described above. Suitable silicones include silicone compounds, as described in detail above. Suitable natural oils include vegetable natural oils, as described in detail above. Suitable thickening polymers include water soluble or dispersible, preferably cationic polymers, as described above. Suitable cationic polymeric materials include cationic guar polymers, cationic cellulose derivatives, cationic potato starch, cationic polyacrylamides. Especially suitable are cross-linked water swellable cationic polymers, such as copolymers of acrylamide with dimethylaminoethyl methacrylate quaternized with methyl chloride, preferably copolymers of about 20 mol-% acrylamide and about mol-80 % dimethyl amino ethyl methacrylate methyl chloride cross-linked with from 450 to 600 ppm of methylene bisacrylamide.

### PREPARATION OF THE HAIR CONDITIONER COMPOSITION

The hair conditioner composition of the invention, as defined herein, can be obtained following a conventional process of mixing the different components, well known by any skilled person. For example, the process comprises the dispersion in hot water, heated at enough temperature to allow a good dispersion, of the quaternary ester ammonium composition as described above, in molten state. In a preferred process of obtention, further ingredients, if present in the composition, are added to the water previous to the dispersion of the composition, once the composition is dispersed in water or blended with the composition in molten state and then added to water. Dispersion temperature is in the range between 40-80°C. The aqueous dispersion is homogeneized by stirring and then cooled down until reaching temperature of 20-25°C. Additional ingredients such perfume and preservatives are added afterwards at a temperature between 30-40°C.

### METHOD FOR CONDITIONING HAIR

A method of conditioning human hair, wherein the hair conditioner composition of the present invention is applied to wet hair and further rinsed from the hair with water, or alternatively be left on hair as leave-on conditioner, is also a part of the invention.

In a preferred embodiment of the invention, the hair conditioner composition of the invention is applied to the wet hair and subsequently left on the hair, as a leave-on hair conditioner.

The use of the hair conditioner composition of the present invention to provide a conditioning effect to the hair is also a part of the invention.

The present invention provides unexpected and advantageous technical effects.

The present invention provides compositions, as defined herein, able to comply with the requirements imposed on them, such as high softening performance, hydrolysis stability in aqueous dispersions with little change in dispersion viscosity, storage stability, appropriate provision of lubricity in fibers, suitable handling and processing in liquid state, good odour, and/or suitable compatibility with other components including perfumes, especially in the the context of fabric softener applications. The present invention provides compositions, as defined herein, with good performance, e.g., in terms of combing force, shine, volume, body, elasticity, manageability, and/or appropriate lubricity of hair fibers and antistatic effect, especially the context of hair conditioner applications.

Compositions according to the invention can exhibit particularly improved viscosity stability upon prolonged storage. Destabilization of a formulation can be preceded by a (i) reduction of the viscosity until the formulation is separated; or (ii) by a drastic increase of the viscosity (at least +400%), making the formulation unpourable from the bottle. However, compositions according to the present invention exhibit no reduction of the viscosity, and no increase or only small increase of viscosity upon storage at 40°C for 90 days, thus demonstrating the improved maintenance of stable viscosity.

Compositions according to the invention can provide particularly good fabric lubricity. Fabrics treated with a composition according to the present invention require a decreased force to be pulled through the cylinders of a testing device (i.e., exhibit decreased friction), thus demonstrating that the present invention improves lubricity.

Compositions according to the invention can provide particularly good hair combability. Hair treated with a composition according to the invention exhibits decreased combing force, thus demonstrating that the present invention improves combability.

### EXAMPLES

The following examples are given in order to provide a person skilled in the art with a sufficiently clear and complete explanation of the present invention, but should not be considered as limiting of the essential aspects of its subject, as set out in the preceding portions of this description.

The first part of the Examples section refers to the preparation of the quaternary ester ammonium compositions according to the invention.

The second part refers to the preparation of a fabric softener composition according to the invention and the determination of the viscosity and the lubricity of the composition.

The third part refers to the preparation of a hair conditioner composition according to the invention and the determination of the combing force of the composition.

### 1. Quaternary ester ammonium compositions

### Preparation of Composition A (comparative)

### Esterification:

750.0 grams (2.76 mol) of tallow fatty acid and 250.0 grams (0.92 mol) of hydrogenated tallow fatty acid were introduced in an inert atmosphere into a stainless steel reactor together with 332.0 g (2.23 mol) of triethanolamine, which were added with stirring. The mixture was heated for at least 4 hours at 160-180°C in order to remove water from the reaction. The final point of the reaction is monitored by an acid value assay until the value was below 2 mg KOH/g.

A yellowish liquid product from the esterification was obtained, comprising a mixture of unesterified fatty acids, mono-, di- and triesterified triethanolamine and unreacted triethanolamine.

### Quaternization:

153.09 grams of isopropyl alcohol were added with stirring to 1140.27 grams of the product from esterification step (containing 2.00 mol of esterified product). Then 237.50 grams (1.88 mol) of dimethyl sulphate were added with stirring at a temperature of 50-90°C. After four hours of digestion, the virtually complete absence of amine value was verified by acid/base assay. 1530.86 grams of the final product (Composition A) was obtained.

### Preparation of Composition B (according to the invention)

### Esterification:

716.9 grams (2.63 mol) of tallow fatty acid and 239.0 grams (0.88 mol) of hydrogenated tallow fatty acid were introduced in an inert atmosphere into a stainless steel reactor, together with 101.1g (0.37 mol) of stearyl alcohol and 298.8g (2.00 mol) of triethanolamine, which were added with stirring. The mixture was heated for at least 4 hours at 160-180°C in order to remove water from the reaction. The final point of the reaction is monitored by an acid value assay until the value was below 2 mg KOH/g.

A yellowish liquid product from the esterification was obtained, comprising a mixture of unesterified fatty acids, mono-, di- and triesterified triethanolamine together with esterified stearyl alcohol and unreacted triethanolamine and stearyl alcohol.

### Quaternization:

111.8 grams of isopropyl myristate were added with stirring to 1163.3 grams of the product from esterification step (containing 1.80 mol of esterified product). Then 216.2 grams (1.71 mol) of dimethyl sulphate were added with stirring at a temperature of 50-90°C. After four hours of digestion, 147.5g of 2-propanol were added with stirring. The virtually complete absence of amine value was verified by acid/base assay. 1638.8 grams of the final product (Composition B) was obtained.

Table 1 below summarizes content of components (b1), (b2) and (c) in Composition B.

**Table 1**

| **Component** | **Content (% wt.)** |
|---|---|
| Component (b1) | 1.8 |
| Component (b2) | 7.5 |
| Component (c) | 0.7 |

### 2. Fabric softener compositions

### Preparation of the softener compositions

Softener compositions were made by dispersing the quaternary ester ammonium composition A or B described above into water. Aqueous dispersions contain 4.5 % of the quaternary ester ammonium composition and 0.2 % of a thickening polymer (i.e. FLOSOFT 222 manufactured by SNF) in water.

The dispersion process consists of heating deionized water at 42°C in a jacketed glass reactor, adding the quaternary ester ammonium composition of interest in the molten state (heated 5 to 10°C above the melting point) and homogenizing the dispersion at a rate of 500 rpm during 20 min, with a helix with four blades stirrer, then adding the thickening polymer while stirring, until complete incorporation. The aqueous dispersion is finally cooled down up to 20-25°C, at a rate of 1.0°C/min, maintaining the agitation at 150 rpm.

### Evaluation of stability and lubricity of fabric softener compositions

### Viscosity stability

Initial viscosity of the aqueous dispersions was determined at 20°C, 24 hours after preparation, with a Brookfield viscometer model LV, using a spindle number 2 at 60 rpm.

Evolution of the viscosity was measured using same conditions following formula stability for at least 3 months on samples stored at room temperature and at 40°C.

For the comparative fabric softener composition (comprising Composition A), the variation of viscosity after 90 days, at 40°C, is -42%, whereas the variation of viscosity after 90 days, at 40°C of the fabric softener composition according to the invention (comprising Composition B) is +23%. Destabilization of a softener formulation can be preceded by a reduction of the viscosity until the formulation is separated or by a drastic increase of the viscosity (at least +400%), making the formulation unpourable from the bottle. In this case, Comparative A shows a negative evolution of the viscosity, leading to separation, whereas the small increase of viscosity of the invention demonstrate the maintenance of stable viscosity of the invention upon storage at 40°C for 90 days.

### Fabric lubricity

Application of softener compositions on cotton fabric (ref. EMPA 211 from EMPA, cotton fabric, percale, bleached, without optical brightener). Previously scoured cotton fabrics were washed at 40 °C with a heavy-duty powder detergent (at a dosage of 2.7% on weight of fabric), rinsed twice and spinning dried. Wet towels were treated for 10 minutes at 25°C with the corresponding aqueous dispersions diluted in water to provide a dosage of 0.12% quaternary ester ammonium compositions on weight of fabric, for a bath ratio of 1/10. Treated cotton fabrics were finally let dry by hanging, and left still for 24 hours under controlled atmospheric conditions (60% RH and 20°C). A blank sample (wet cotton fabric not treated with an aqueous dispersion of the softener composition) was also prepared and further measured.

Measurements of cotton fabrics lubricity were done using a universal testing machine Instron 5543 with the Aqualon SLT accessory in the stationary mode at 20 °C and 60 % RH. A cotton fabric piece of 25x8 cm is folded 4 times to fit into the Aqualon SLT accessory (J. Cosmet. Sci. 2009, 60, 135-141). Lubricity is measured as the force required to pull the fabric through the cylinders from 5 to 60 mm. Results are expressed in kgf·mm.

The friction coefficient of the Invention B decreases by -17% compared to the friction coefficient of the untreated sample (blank).

### 3. Hair conditioner compositions

### Preparation of the hair conditioner compositions

Hair conditioners were made by dispersing quaternary ester ammonium composition A or B described above into water. Aqueous dispersions contain 4.5 % of the quaternary ester ammonium composition in water.

The dispersion process consists of heating deionized water at 42°C in a jacketed glass reactor, adding the quaternary ester ammonium composition of interest in the molten state (heated 5 to 10°C above the melting point) and homogenizing the dispersion with a helix with four blades stirrer at a rate of 500 rpm during 20 min. The aqueous dispersion is finally cooled down up to 20-25 °C, at a rate of 1.0 °C/min, maintaining the agitation at 150 rpm.

### Evaluation of combing force of hair conditioner compositions

Application of hair conditioners on hair tress (ref. 826500 from Kerling, European natural hair, adhesive tress tight, glued on one side, total length 150 mm, free length 130 mm, width 20 mm, weight 1.8 ± 0.2 g). Dry hair tresses were treated during 5 minutes at 25°C with the corresponding aqueous dispersions diluted in water to provide a dosage of 0.12 % quaternary ester ammonium containing compositions on weight of hair, for a bath ratio of 1/5. Exceeding water is then removed by squeezing wet hair tresses between fingers, leaving hair tresses ready to be measured in wet condition. A blank sample (hair tress not treated with the hair conditioner) was also prepared and further measured.

Measurements of the combing force were done using a universal testing machine Instron 5543 with the combing accessory at 20°C and 60% RH. Wet hair tress is attached to the slot of the lift mechanism and hair is properly distributed through all comb teeth, as explained in J. Soc. Cosmet. Chem. 1973, 24, 773-782. The force required to pull the hair tress through the comb is measured 10 times. Results are expressed in kgf.

Combing force of the hair conditioner composition according to the invention (comprising Composition B) is 345 kgf, whereas combing force of blank sample is higher than 1000 kgf, which can be considered as uncombable.

## Claims

1. A composition comprising:
i. component (a) which comprises at least one or more quaternary mono-, di-, or tri-ester ammonium compounds; and
ii. component (b), said component being a fatty acid ester mixture, comprising:
- at least a component (b1) being a fatty acid ester or a mixture of fatty acid esters of formula (II),
R₅-COO-R₆ Formula (II)
wherein R₅ represents a fatty acid moiety being a linear alkyl containing 5 to 23 carbon atoms, or a linear alkenyl group containing 5 to 23 carbon atoms and from 1 to 3 double bonds; R₆ represents an alkyl or alkenyl group derived from a linear alcohol containing 6 to 24 carbon atoms; and
- at least a component (b2) being a fatty acid ester or a mixture of fatty acid esters of formula (III),
R₇-COO-R₈ Formula (III)
wherein R7 represents a fatty acid moiety being a linear or branched alkyl containing 5 to 23 carbon atoms, or a linear or branched alkenyl containing 5 to 23 carbon atoms and from 1 to 3 double bonds; R8 represents an alkyl or alkenyl group derived from a linear or branched alcohol containing 3 to 24 carbon atoms; with the proviso that at least one of R₇ and R₈ represents a branched alkyl or alkenyl group; and
iii. component (c), said component being a fatty alcohol or a mixture of fatty alcohols of formula (IV),
R₉-O-(CH₂CH₂O)ₗ-(CH₂CHR₁₀O)ₖ-H Formula (IV)
wherein R₉ represents an alkyl or alkenyl group containing 6 to 24 carbon atoms, R₁₀ represents an alkyl group containing 1 to 4 carbon atoms; l represents a number within the range of 0 to 10, k represents a number within the range of 0 to 10, and the sum of l+k represents the average alkoxylation degree which corresponds to a number from 0 to 20, preferably from 0 to 10, more preferably from 0 to 5, and
wherein the composition comprises, in the indicated amounts expressed as percentage by weight with respect to the total weight of the composition:
0.6 to 20.0% of component (b1),
1.0 to 29.0% of component (b2), and
0.4 to 9.0% of component (c).

2. The composition according to claim 1, wherein the weight ratio between the component (b1) and (b2), that is (b1): (b2), is 70:30 to 5:95; and/or
wherein the weight ratio of the sum of components (b1) and (c) to component (b2), that is ((b1)+(c)):(b2), is 75:25 to 5:95.

3. A process for producing the composition as defined in claim 1 or 2, comprising the steps:
i. esterification step, wherein a fatty acid, a methyl ester or a triglyceride thereof is reacted with an alkanolamine to obtain a mixture containing an esteramine; and
ii. quaternization step, wherein the mixture obtained after the esterification step is reacted with an alkylating agent,
wherein the component (b1) present in the composition is intentionally added and/or generated in situ in the esterification step, after the esterification step, in the quaternization step or after the quaternization step; and
wherein the component (b2) present in the composition is intentionally added after the esterification step, in the quaternization step or after the quaternization step.

4. The process according to claim 3, wherein the component (b1) is generated in situ by reaction with component (c) which is added in the esterification step, after the esterification step, in the quaternization step or after the quaternization step; and/or
wherein component (c) present in the composition is intentionally added in the esterification step, after the esterification step, in the quaternization step or after the quaternization step.

5. A fabric softener composition comprising a composition according to claim 1 or 2 or a composition obtained by the process as defined in claim 3 or 4, and water.

6. The fabric softener composition according to claim 5, further comprising:
- from 0.01 to 3% of thickening polymer(s); and/or
- from 0 to 2% of electrolyte concentration aid(s); and/or
- from 0.01 to 5% of perfume(s);and/or
- from 0 to 5% of non-ionic surfactant(s),
wherein indicated amounts are expressed as percentage by weight with respect to the total weight of the composition.

7. A process for producing the fabric softener composition as defined in claim 5 or 6, comprising the steps:
i. adding the composition as defined in claim 1 or 2, or the composition obtained by the process as defined in claim 3 or 4, in a molten state to water;
ii. stirring to obtain a homogeneous dispersion.

8. The process according to claim 7, further comprising the steps:
iii. cooling down; and
iv. optionally mixing with the further components, wherein the further components can be added to the aqueous phase before or after the dispersion of the composition and before or after any one of steps i) to iii).

9. Use of the fabric softener composition as defined in claim 5 or 6 to soften and condition textiles or fabrics.

10. A method to soften and condition textiles or fabrics, comprising the step of applying a composition as defined in claim 5 or 6, or obtained by the method as defined in claim 7 or 8, to textiles or fabrics.

11. A hair conditioner composition comprising a composition according to claim 1 or 2, or obtained by the process as defined in claim 3 or 4, and water.

12. The hair conditioner composition according to claim 11, further comprising:
- from 0.5 to 5% of emulsifiers; and/or
- from 0.2 to 3% of perfume; and/or
- from 0 to 5% of silicones; and/or
- from 0 to 2% of thickening polymers; and/or
- from 0 to 5% of natural oils,
wherein indicated amounts are expressed as percentage by weight with respect to the total weight of the composition.

13. A process for producing the hair conditioner composition as defined in claim 11 or 12, comprising the step of dispersing in water, at room temperature, of a composition as defined in claim 1 or 2 or obtained by the process as defined in claim 3 or 4.

14. Use of the hair conditioner composition as defined in claim 11 or 12 for the conditioning treatment of hair.

15. A method of conditioning hair, comprising the step of applying the composition as defined in claim 11 or 12, or obtained by the process as defined in claim 13, to hair.

## Patentansprüche

1. Zusammensetzung umfassend:
i. Komponente (a), welche mindestens eine oder mehrere quaternäre Mono-, Di- oder Triesterammoniumverbindungen umfasst; und
ii. Komponente (b), wobei die genannte Komponente eine Fettsäureestermischung ist, umfassend:
- mindestens eine Komponente (b1), welche ein Fettsäureester oder eine Mischung aus Fettsäureestern der Formel (II) ist,
R₅-COO-R₆ Formel (II)
in welcher R₅ für einen Fettsäurerest steht, welcher ein lineares Alkyl enthaltend 5 bis 23 Kohlenstoffatome, oder eine lineare Alkenylgruppe enthaltend 5 bis 23 Kohlenstoffatome und von 1 bis 3 Doppelbindungen ist; R₆ für eine Alkyl- oder Alkenylgruppe abgeleitet von einem linearen Alkohol enthaltend 6 bis 24 Kohlenstoffatome steht; und
- mindestens eine Komponente (b2), welche ein Fettsäureester oder eine Mischung aus Fettsäureestern der Formel (III) ist,
R₇-COO-R₈ Formel (III)
in welcher R₇ für einen Fettsäurerest steht, welcher ein lineares oder verzweigtes Alkyl enthaltend 5 bis 23 Kohlenstoffatome, oder ein lineares oder verzweigtes Alkenyl enthaltend 5 bis 23 Kohlenstoffatome und von 1 bis 3 Doppelbindungen ist; R₈ für eine Alkyl- oder Alkenylgruppe abgeleitet von einem linearen oder verzweigten Alkohol enthaltend 3 bis 24 Kohlenstoffatome steht; mit der Maßgabe, dass mindestens einer von R₇ und R₈ für eine verzweigte Alkyl- oder Alkenylgruppe steht; und
iii. Komponente (c), wobei die genannte Komponente ein Fettalkohol oder eine Mischung aus Fettalkoholen der Formel (IV) ist,
R₉-O-(CH₂CH₂O)ₗ-(CH₂CHR₁₀O)ₖ-H Formel (IV)
in welcher R₉ für eine Alkyl- oder Alkenylgruppe enthaltend 6 bis 24 Kohlenstoffatome steht, R₁₀ für eine Alkylgruppe enthaltend 1 bis 4 Kohlenstoffatome steht; l für eine Nummer im Bereich von 0 bis 10 steht, k für eine Nummer im Bereich von 0 bis 10 steht, und die Summe von I+k für den durchschnittlichen Alkoxylierungsgrad steht, welcher einer Nummer von 0 bis 20, vorzugsweise von 0 bis 10, weiter vorzugsweise von 0 bis 5 entspricht, und
wobei die Zusammensetzung, in den angegebenen Mengen ausgedrückt als Gewichtsprozent in Bezug auf das Gesamtgewicht der Zusammensetzung, Folgendes umfasst:
0,6 bis 20,0 % Komponente (b1),
1,0 bis 29,0 % Komponente (b2), und
0,4 bis 9,0 % Komponente (c).

2. Zusammensetzung nach Anspruch 1, wobei das Gewichtsverhältnis zwischen der Komponente (b1) und (b2), das heißt (b1):(b2), 70:30 bis 5:95 ist; und/oder
wobei das Gewichtsverhältnis der Summe von Komponenten (b1) und (c) zu Komponente (b2), das heißt ((b1)+(c)):(b2), 75:25 bis 5:95 ist.

3. Prozess zur Herstellung der Zusammensetzung nach Anspruch 1 oder 2, umfassend die folgenden Schritte:
i. einen Veresterungsschritt, in welchem eine Fettsäure, ein Methylester oder ein Triglycerid derselben mit einem Alkanolamin umgesetzt wird, um eine Mischung enthaltend ein Esteramin zu erhalten; und
ii. einen Quaternisierungsschritt, in welchem die nach dem Veresterungsschritt erhaltene Mischung mit einem Alkylierungsmittel umgesetzt wird,
wobei die in der Zusammensetzung vorhandene Komponente (b1) im Veresterungsschritt, nach dem Veresterungsschritt, im Quaternisierungsschritt oder nach dem Quaternisierungsschritt absichtlich hinzugefügt und/oder *in situ* erzeugt wird; und
wobei die in der Zusammensetzung vorhandene Komponente (b2) nach dem Veresterungsschritt, im Quaternisierungsschritt oder nach dem Quaternisierungsschritt absichtlich hinzugefügt wird.

4. Prozess nach Anspruch 3, wobei die Komponente (b1) mittels der Reaktion mit Komponente (c), welche im Veresterungsschritt, nach dem Veresterungsschritt, im Quaternisierungsschritt oder nach dem Quaternisierungsschritt hinzugefügt wird, *in situ* erzeugt wird; und/oder
wobei die in der Zusammensetzung vorhandene Komponente (c) im Veresterungsschritt, nach dem Veresterungsschritt, im Quaternisierungsschritt oder nach dem Quaternisierungsschritt absichtlich hinzugefügt wird.

5. Weichspülerzusammensetzung umfassend eine Zusammensetzung nach Anspruch 1 oder 2 oder eine durch den Prozess nach Anspruch 3 oder 4 erhaltene Zusammensetzung, und Wasser.

6. Weichspülerzusammensetzung nach Anspruch 5, zusätzlich umfassend:
- von 0,01 bis 3 % Verdickungspolymer(e); und/oder
- von 0 bis 2 % Elektrolytkonzentrierungshilfsmittel; und/oder
- von 0,01 bis 5 % Duftstoff(e); und/oder
- von 0 bis 5 % nichtionisches Tensid/nichtionische Tenside,
wobei die angegebenen Mengen als Gewichtsprozent in Bezug auf das Gesamtgewicht der Zusammensetzung ausgedrückt sind.

7. Prozess zur Herstellung der Weichspülerzusammensetzung nach Anspruch 5 oder 6, umfassend die folgenden Schritte:
i. das Hinzufügen der Zusammensetzung nach Anspruch 1 oder 2, oder der durch den Prozess nach Anspruch 3 oder 4 erhaltenen Zusammensetzung, in einem geschmolzenen Zustand zu Wasser;
ii. das Rühren, um eine homogene Dispersion zu erhalten.

8. Prozess nach Anspruch 7, zusätzlich umfassend die folgenden Schritte:
iii. das Abkühlen; und
iv. wahlweise das Mischen mit den zusätzlichen Komponenten, wobei die zusätzlichen Komponenten zur wässrigen Phase vor oder nach der Dispergierung der Zusammensetzung und vor oder nach einem der Schritte i) bis iii) hinzugefügt werden können.

9. Verwendung der Weichspülerzusammensetzung nach Anspruch 5 oder 6, um Textilien oder Gewebe aufzuweichen und zu konditionieren.

10. Verfahren zum Aufweichen und Konditionieren von Textilien oder Geweben, umfassend den Schritt des Applizierens einer Zusammensetzung nach Anspruch 5 oder 6, oder erhalten durch das Verfahren nach Anspruch 7 oder 8, auf Textilien oder Gewebe.

11. Haarspülungzusammensetzung, umfassend eine Zusammensetzung nach Anspruch 1 oder 2, oder erhalten durch den Prozess nach Anspruch 3 oder 4, und Wasser.

12. Haarspülungzusammensetzung nach Anspruch 11, zusätzlich umfassend:
- von 0,5 bis 5 % Emulgiermittel; und/oder
- von 0,2 bis 3 % Duftstoff; und/oder
- von 0 bis 5 % Silikone; und/oder
- von 0 bis 2 % Verdickungspolymere; und/oder
- von 0 bis 5 % natürliche Öle,
wobei die angegebenen Mengen als Gewichtsprozent in Bezug auf das Gesamtgewicht der Zusammensetzung ausgedrückt sind.

13. Prozess zur Herstellung der Haarspülungzusammensetzung nach Anspruch 11 oder 12, umfassend den Schritt des Dispergierens in Wasser, bei Raumtemperatur, einer Zusammensetzung nach Anspruch 1 oder 2 oder erhalten durch den Prozess nach Anspruch 3 oder 4.

14. Verwendung der Haarspülungzusammensetzung nach Anspruch 11 oder 12, für die Konditionierungsbehandlung der Haare.

15. Verfahren für die Konditionierung der Haare, umfassend den Schritt des Applizierens der Zusammensetzung nach Anspruch 11 oder 12, oder erhalten durch den Prozess nach Anspruch 13, auf die Haare.

## Revendications

1. Composition comprenant :
i. un composant (a) qui comprend au moins un ou plusieurs composés d'ammonium mono, di, ou tri-ester quaternaire ; et
ii. un composant (b), ledit composant étant un mélange d'esters d'acides gras, comprenant :
- au moins un composant (b1) qui est un ester d'acide gras ou un mélange de esters d'acides gras de formule (II),
R₅-COO-R₆ Formule (II)
dans laquelle R₅ représente un groupement acide gras qui est un alkyle linéaire contenant 5 à 23 atomes de carbone, ou un groupe alcényle linéaire contenant 5 à 23 atomes de carbone et 1 jusqu'à 3 doubles liaisons ; R₆ représente un groupe alkyle ou alcényle dérivé d'un alcool linéaire contenant 6 à 24 atomes de carbone ; et
- au moins un composant (b2) qui est un ester d'acide gras ou un mélange de esters d'acides gras de formule (III),
R₇-COO-R₈ Formule (III)
dans laquelle R₇ représente un groupement acide gras qui est un alkyle linéaire ou ramifié contenant 5 à 23 atomes de carbone, ou un alcényle linéaire ou ramifié contenant 5 à 23 atomes de carbone et 1 jusqu'à 3 doubles liaisons ; R₈ représente un groupe alkyle ou alcényle dérivé d'un alcool linéaire ou ramifié contenant 3 à 24 atomes de carbone ; à condition qu'au moins l'un parmi R₇ et R₈ représente un groupe alkyle ou alcényle ramifié ; et
iii. un composant (c), ledit composant étant un alcool gras ou un mélange d'alcools gras de formule (IV),
R₉-O-(CH₂CH₂O)ₗ-(CH₂CHR₁₀O)ₖ-H Formule (IV)
dans laquelle R₉ représente un groupe alkyle ou alcényle contenant 6 à 24 atomes de carbone, R₁₀ représente un groupe alkyle contenant 1 à 4 atomes de carbone ; l représente un nombre dans l'intervalle de 0 à 10, k représente un nombre dans l'intervalle de 0 à 10, et la somme de l+k représente le degré d'alcoxylation moyen qui correspond à un nombre de 0 jusqu'à 20, de préférence de 0 jusqu'à 10, plus de préférence de 0 jusqu'à 5, et
dans laquelle la composition comprend, dans les quantités indiquées exprimées en pourcentage en poids par rapport au poids total de la composition :
0,6 à 20,0 % de composant (b1),
1,0 à 29,0 % de composant (b2), et
0,4 à 9,0 % de composant (c).

2. Composition selon la revendication 1, dans laquelle le rapport en poids entre le composant (b1) et (b2), c'est-à-dire (b1) : (b2), est de 70 :30 à 5 :95 ; et/ou
dans laquelle le rapport en poids de la somme des composants (b1) et (c) par rapport au composant (b2), c'est-à-dire ((b1)+(c)) : (b2), est de 75 :25 à 5 :95.

3. Procédé pour produire la composition selon la revendication 1 ou 2, comprenant les étapes suivantes :
i. étape d'esterification, dans laquelle on fait réagir un acide gras, un ester méthylique ou un triglycéride de celui-ci, avec une alcanolamine pour obtenir un mélange contenant une esteramine ; et
ii. étape de quaternisation, dans laquelle on fait réagir le mélange obtenu après l'étape d'esterification avec un agent alkylant,
dans lequel le composant (b1) présent dans la composition est ajouté intentionnellement et/ou généré *in situ* dans l'étape d'esterification, après l'étape d'esterification, dans l'étape de quaternisation ou après l'étape de quaternisation ; et
dans lequel le composant (b2) présent dans la composition est ajouté intentionnellement après l'étape d'esterification, dans l'étape de quaternisation ou après l'étape de quaternisation.

4. Procédé selon la revendication 3, dans lequel le composant (b1) est généré *in situ* par réaction avec le composant (c) qui est ajouté dans l'étape d'esterification, après l'étape d'esterification, dans l'étape de quaternisation ou après l'étape de quaternisation ; et/ou
dans lequel le composant (c) présent dans la composition est ajouté intentionnellement dans l'étape d'esterification, après l'étape d'esterification, dans l'étape de quaternisation ou après l'étape de quaternisation.

5. Composition assouplissante de tissus comprenant une composition selon la revendication 1 ou 2 ou une composition obtenue par biais du procédé selon la revendication 3 ou 4, et de l'eau.

6. Composition assouplissante de tissus selon la revendication 5, comprenant en outre :
- 0,01 jusqu'à 3 % de polymère(s) épaississant(s) ; et/ou
- 0 jusqu'à 2 % d'adjuvant(s) de concentration d'électrolyte ; et/ou
- 0,01 jusqu'à 5 % de parfum(s) ; et/ou
- 0 jusqu'à 5 % de tensioactif(s) non ionique(s),
dans laquelle les quantités indiquées sont exprimées en pourcentage en poids par rapport au poids total de la composition.

7. Procédé pour produire la composition assouplissante de tissus selon la revendication 5 ou 6, comprenant les étapes suivantes :
i. ajouter la composition selon la revendication 1 ou 2, ou la composition obtenue par le biais du procédé selon la revendication 3 ou 4, à l'état fondu à l'eau ;
ii. agiter pour obtenir une dispersion homogène.

8. Procédé selon la revendication 7, comprenant en outre les étapes suivantes :
iii. refroidir ; et
iv. optionnellement mélanger avec les composants additionnels, dans lequel les composants additionnels peuvent être ajoutés à la phase aqueuse avant ou après la dispersion de la composition et avant ou après l'une quelconque des étapes i) à iii).

9. Utilisation de la composition assouplissante de tissus selon la revendication 5 ou 6 pour assouplir et conditionner des textiles ou des tissus.

10. Méthode pour assouplir et conditionner des textiles ou des tissus, comprenant l'étape consistant à appliquer une composition selon la revendication 5 ou 6, ou obtenue par le biais de la méthode selon la revendication 7 ou 8, à des textiles ou des tissus.

11. Composition après-shampooing comprenant une composition selon la revendication 1 ou 2, ou obtenue par le biais du procédé selon la revendication 3 ou 4, et de l'eau.

12. Composition après-shampooing selon la revendication 11, comprenant en outre :
- 0,5 jusqu'à 5 % d'émulsifiants ; et/ou
- 0,2 jusqu'à 3 % de parfum ; et/ou
- 0 jusqu'à 5 % de silicones ; et/ou
- 0 jusqu'à 2 % de polymères épaississants ; et/ou
- 0 jusqu'à 5 % d'huiles naturelles,
dans laquelle les quantités indiquées sont exprimées en pourcentage en poids par rapport au poids total de la composition.

13. Procédé pour produire la composition après-shampooing selon la revendication 11 ou 12, comprenant l'étape consistant à disperser dans l'eau, à température ambiante, une composition selon la revendication 1 ou 2 ou obtenue par le biais du procédé selon la revendication 3 ou 4.

14. Utilisation de la composition après-shampooing selon la revendication 11 ou 12, pour le traitement de conditionnement des cheveux.

15. Méthode de conditionnement des cheveux, comprenant l'étape consistant à appliquer la composition selon la revendication 11 ou 12, ou obtenue par le biais du procédé selon la revendication 13, aux cheveux.
